# EUROPEAN PATENT SPECIFICATION

(11) **EP 1 742 665 B1**
(45) Date of publication and mention of the grant of the patent: **02.11.2011**
(21) Application number: 05739818.2
(22) Date of filing: 06.05.2005
(51) Int. Cl.: A61K 47/34, C08G 63/91

(54) **DELIVERY SYSTEM FOR BIOACTIVE AGENTS ON THE BASIS OF A POLYMERIC DRUG CARRIER COMPRISING AN AMPHIPHILIC BLOCK POLYMER AND A POLYLACTICACID DERIVATIVE**
ABGABESYSTEM FÜR BIOLOGISCH WIRKSAME MITTEL AUF BASIS EINES POLYMEREN ARZNEITRÄGERS MIT EINEM AMPIPHILEN BLOCKPOLYMER UND EINEM POLYMILCHSÄURE-DERIVAT
SYSTEME D'ADMINISTRATION POUR AGENTS BIOACTIFS BASE SUR UN EXCIPIENT MEDICAMENTEUX POLYMERE COMPORTANT UN POLYMERE SEQUENCE AMPHIPHILE ET UN DERIVE D'ACIDE POLYLACTIQUE

(30) Priority: 06.05.2004 US 568945 P
(43) Date of publication of application: 17.01.2007
(73) Proprietor: Samyang Corporation, Jongro-gu, Seoul 110-470 (KR)
(72) Inventor: YI, Yil-Woong, Daejeon-city 305-390 (KR); LEE, Sa-Won, Daejeon-City 305-345 (KR); YU, Jeong-Il, Daejeon-city 305-345 (KR); CHANG, Dong-Hoon, Seoul 138-121 (KR); SEO, Min-Hyo, Daejeon-city 302-122 (KR); KANG, Hye-Won, Daejeon-city 305-348 (KR); KIM, Jae-Hong, Daejeon-city 305-311 (KR)
(74) Representative: Chalk, Anthony John
(86) International application number: PCT/KR2005/001330
(87) International publication number: WO 2005/107813

(56) References cited:
- WO-A2-03/059382

## Description

### CROSS REFERENCE TO RELATED APPLICATION

This application claims priority to and the benefit of US Patent Application No. 60/568,945 in the United States Patent and Trademark Office on May 6, 2004, the entire content of which is incorporated hereinto by reference.

### FIELD OF THE INVENTION

This present invention relates to a delivery system and a method for the intracellular delivery of bioactive agents using polymeric drug carriers. More particularly, it relates to a method for the intracellular delivery of bioactive agents using polymeric drug carriers formed from (a) an amphiphilic block copolymer which is comprised of a hydrophilic block and a hydrophobic block, wherein said hydrophobic block has a terminal hydroxyl group that is substituted with a tocopherol or cholesterol group, and (b) a polylactic acid derivative having at least one terminal carboxyl group at the end of the polymer.

### BACKGROUND OF THE INVENTION

In order to achieve the desired therapeutic effect of a bioactive agent, an appropriate amount of the administered drug should enter the target cells in a body. In order to increase the cellular internalization of a drug, an appropriate concentration of the drug should be maintained for a desired time period in the target tissue; furthermore, the drug should enter the target cells in the tissue. A high drug concentration in a tissue can be achieved by a formulation exhibiting a long blood circulation time. Therefore, great effort has been made to develop drug delivery systems by the use of nanoparticulate drug carriers, including liposome and polymeric micelles, having long circulation times.

Many approaches have been suggested for enhancing the intracellular uptake of bioactive agents. Delivery vehicles such as liposomes have also been described for use in the intracellular delivery of bioactive agents such as oligonucleotides (Felgner, et al., U.S. Pat. No. 5,264,618 (1993); Eppstein, et al., U.S. Pat. No. 4,897,355 (1990); and Wang, et al., Proc. Nat. Acad. Sci. 84: 7851-7855 (1987); U.S. Pat. No. 5,759,519 (1998), WO2005035606, WO03033592. The use of liposomes as drug carriers, however, is limited due to such problems as low entrapment efficiency, drug instability, rapid drug leakage, and poor storage stability. Small molecular surfactant micelles are easily dissociated when they are diluted with body fluids after being administered into the body; so it is difficult for them to perform their role as drug carriers.

In recent years, efforts have been made for the preparation, characterization and pharmaceutical application of polymeric micelles. These were well reviewed by V. Torchilin in Journal of Controlled Release 73(2001) pp.137-172. Polymeric micelles are characterized by a core-shell structure in aqueous media which results from the amphiphilic block copolymers having hydrophobic (core) and hydrophilic (shell) segments. A poorly water soluble drug is entrapped within the hydrophobic core of the micelle. There has been considerable research into the development of A-B, A-B-A, or B-A-B block copolymers having a hydrophilic A block and a hydrophobic B block. For use as a drug carrier, it is preferred that the hydrophobic B(inner micelle core block) comprises a biodegradable polymer such as poly-DL-lactide, poly- ε -caprolactone or poly(γ-benzyl-L-aspartate) and that the hydrophilic A (outer micelle shell block) be a polymer which is capable of interacting with plasma proteins and cell membranes, such as polyethylene glycol(PEG).

Polymeric micelles provide attractive characteristics in that they can avoid uptake of the drug by the reticuloendothelial system (RES) or the mononuclear phagocyte system (MPS) in vivo, and hence, they can circulate in the blood for a long period of time. This advantage comes from the structure of a micelle. The hydrophilic portions of an amphiphilic block copolymer form the outer shell and are exposed to body fluid, and hence, effectively protect the micelles from interactions with the cell membranes and plasma proteins in the blood [V. Torchilin et al., Advanced Drug Delivery Reviews 16(1995) pp.141-155].

R. Savic et al. showed experimental evidence that micelles formed from poly(caprolactone)-b-poly(ethylene oxide) block copolymers can deliver a bioactive agent into living cells by using micelles with tetramethylrhodamine-5-carbonyl azide(TMRCA) covalently attached to the PCL end of the polymer [R. Savic et al., Sceince 300(2003) pp.615-618]. However, the fluorescent micelles were detected only in the cytoplasmic but not in the nuclear compartment and thus bioactive agents such as DNA binding anticancer drugs are not appropriate for use with the micelles.

In view of the foregoing, development of a polymeric micelle or nanoparticle which can deliver bioactive agents into the targeted cells is desired. Thus, the present invention provides a method for the targeted intracellular delivery of bioactive agents using a polymeric micelle or nanoparticle drug carrier.

### SUMMARY OF THE INVENTION

It is an object of the present invention to provide a method for the intracellular delivery of bioactive agents using polymeric drug carriers formed from compositions comprising (a) an amphiphilic block copolymer which is comprised of a hydrophilic block and a hydrophobic block, wherein said hydrophobic block has a terminal hydroxyl group that is substituted with a tocopherol or cholesterol group, and (b) a polylactic acid derivative having at least one terminal carboxyl group at the end of the polymer. Optionally, 0.01 to 10 equivalents of a di- or tri-valent metal ion is bound to 1 equivalent of the carboxyl terminal group of said polylactic acid derivative.

It is another object of the present invention to a delivery system for the intracellular delivery of bioactive agents comprising bioactive agents and a polymeric drug carrier with said bioactive agents entrapped therein in the aqueous solution, wherein the polymeric drug carrier is prepared by a polymeric composition comprising (a) an amphiphilic block copolymer consisting of a hydrophilic block and a hydrophobic block in which said hydrophobic block has a terminal hydroxyl group that is substituted with a tocopherol orcholesterol group and (b) a polylactic acid derivative having at least one carboxyl group at the end of the polymer; and wherein said bioactive agents entrapped in the polymeric drug carrier are allowed to be delivered into a cell in a greater quantity when the drug carrier contact with said cell. It is another object of the present invention to a composition for the intracellular delivery of bioactive agents comprising bioactive agents and a polymeric drug carrier with said bioactive agents entrapped therein in the aqueous solution, wherein the polymeric drug carrier is prepared by a polymeric composition comprising (a) an amphiphilic block copolymer consisting of a hydrophilic block and a hydrophobic block in which said hydrophobic block has a terminal hydroxyl group that is substituted with a tocopherol orcholesterol group and (b) a polylactic acid derivative having at least one carboxyl group at the end of the polymer; and wherein said bioactive agents entrapped in the polymeric drug carrier are allowed to be delivered into a cell in a greater quantity when the drug carrier contact with said cell.

The present invention relates to polymeric drug carriers which can deliver a bioactive agent into a targeted cell.

### BRIEF DESCRIPTION OF THE DRAWINGS

Fig. 1 is a schematic diagram of the cellular internalization of a bioactive agent from the body fluid after administration of the composition of the present invention.
Fig. 2A shows the number of cells in which the drug is internalized after treatment of doxorubicin-sensitive cells(MES-SA) with the drug compositions.
Fig. 2B shows the number of cells in which the drug is internalized after treatment of doxorubicin-resistant cells(MES-SA/Dx-5) with the drug compositions.
Fig. 3A shows the fluorescence intensity detected by FACS after treatment of doxorubicin-sensitive cells(MES-SA) with the drug compositions.
Fig. 3B shows the fluorescence intensity detected by FACS after treatment of doxorubicin-resistant cells(MES-SA/Dx-5) with the drug compositions.
Fig. 4A shows confocal microscopic images obtained 2 hours after treatment of doxorubicin-sensitive cells(MES-SA) with the doxorubicin-containing composition (composition 1, right side) and a conventional solution formulation(left side).
Fig. 4B shows confocal microscopic images obtained 8 hours after treatment of doxorubicin-sensitive cells(MES-SA) with the doxorubicin-containing composition (composition 1) and a conventional solution formulation.
Fig. 4C shows confocal microscopic images obtained 2 hours after treatment of doxorubicin-resistant cells(MES-SA/Dx-5) with the doxorubicin-containing composition (composition 1) and a conventional solution formulation.
Fig. 4D shows confocal microscopic images obtained 8 hours after treatment of doxorubicin-resistant cells(MES-SA/Dx-5) with the doxorubicin-containing composition (composition 1) and a conventional solution formulation.
Fig. 4E shows confocal microscopic images obtained 2 hours after treatment of epirubicin-sensitive cells(MCF-7) with the epirubicin-containing composition (composition 6) and conventional solution formulation.
Fig. 4F shows confocal microscopic images obtained 8 hours after treatment of epirubicin-sensitive cells(MCF-7) with the epirubicin-containing composition (composition 6) and a conventional solution formulation.
Fig. 4G shows confocal microscopic images obtained 2 hours after treatment of epirubicin-resistant cells(MCF-7/ADR) with the epirubicin-containing composition (composition 6) and a conventional solution formulation.
Fig. 4H shows confocal microscopic images obtained 8 hours after treatment of a epirubicin-resistant cells(MCF-7/ADR) with the epirubicin-containing composition (composition 6) and a conventional solution formulation.
Fig. 5A shows the cell viability after treatment of doxorubicin-sensitive cells(MES-SA) with the drug compositions (0.1 µg/ml).
Fig. 5B shows the cell viability after treatment of doxorubicin-resistant cells(MES-SA/Dx-5) with the drug compositions (1.0 µg/ml).
Fig. 6 shows drug concentration in blood plasma with time after intravenous administration of the drug compositions in rats.

### DETAILED DESCRIPTION OF THE INVENTION

Before the present invention is disclosed and described, it should be understood that this invention is not limited to the particular configurations, process steps, and materials disclosed herein, and such configurations, process steps, and materials may be varied. It should be also understood that the terminology employed herein is used for the purpose of describing particular embodiments only and is not intended to limit the scope of the present invention which will be limited only by the appended claims and equivalents thereof.

It must be noted that, as used in this specification and the appended claims, the singular forms "a," "an," and "the" include plural referents unless the context clearly dictates otherwise. Thus, for example, reference to a polymer containing "a terminal group" includes reference to two or more such groups.

In describing and claiming the present invention, the following terminology will be used in accordance with the definitions set out below.

As used herein, the term "bioactive agent" means an organic compound or drug which has a desirable biological activity or function, i.e. a biological effect or pharmacological effect, in vivo. For example, bioactive agent consisting of therapeutic agents may alter cellular functions, such as gene function. Alternatively, bioactive agents consisting of diagnostic agents, such as magnetic resonance imaging ("MRI") or computerized tomography ("CT") agents, have the biological function of enhancing the diagnostic images of tissues and/or organs.

As used herein, the term "biodegradable" or "biodegradation" is defined as the conversion of materials into less complex intermediates or end products by solubilization hydrolysis, or by the action of biologically formed entities which can be enzymes or other products of the organism.

As used herein, the term "biocompatible" means materials or the intermediates or end products of materials formed by solubilization hydrolysis, or by the action of biologically formed entities which can be enzymes or other products of the organism and which cause no adverse effects on the organisms.

"Poly(lactide)" or "PLA" shall mean a polymer derived from the condensation of lactic acid or by the ring opening polymerization of lactide. The terms "lactide" and "lactate" are used interchangeably.

Reference will now be made to the exemplary embodiments and specific language will be used herein to describe the same. It will nevertheless be understood that no limitation of the scope of the invention is thereby intended. Alterations and further modifications of the inventive features illustrated herein, and additional applications of the principles of the invention as illustrated herein, which would occur to one skilled in the relevant art and having possession of this disclosure, are to be considered within the scope of the invention.

The present invention provides a method for the intracellular delivery of bioactive agents using polymeric drug carriers formed from polymeric compositions comprising an amphiphilic block copolymer which is comprised of a hydrophilic block and a hydrophobic block, wherein said hydrophobic block has a terminal hydroxyl group that is substituted with a tocopherol or cholesterol group, and a polylactic acid derivative having at least one terminal carboxyl group at the end of the polymer.

The present invention also provides a method for the intracellular delivery of bioactive agents using drug carriers formed from polymeric compositions comprising an amphiphilic block copolymer which comprised of a hydrophilic block and a hydrophobic block, wherein said hydrophobic block has a terminal hydroxyl group that is substituted with a tocopherol succinic acid or cholesterol succinic acid group, a polylactic acid derivative having at least one terminal carboxyl group at the end of the polymer and 0.01 to 10 equivalents of a di- or tri-valent metal ion with respect to 1 equivalent of the carboxyl terminal group of the polylactic acid derivative.

The present invention further provides a delivery system for the intracellular delivery of bioactive agents comprising bioactive agents and a polymeric drug carrier with said bioactive agents entrapped therein in the aqueous solution, wherein the polymeric drug carrier is prepared by a polymeric composition comprising (a) an amphiphilic block copolymer consisting of a hydrophilic block and a hydrophobic block in which said hydrophobic block has a terminal hydroxyl group that is substituted with a tocopherol orcholesterol group and (b) a polylactic acid derivative having at least one carboxyl group at the end of the polymer; and wherein said bioactive agents entrapped in the polymeric drug carrier are allowed to be delivered into a cell in a greater quantity when the drug carrier contact with said cell.

In an embodiment of the present invention, the bioactive agents are allowed to be delivered into a cell in a greater quantity, and preferably, in more efficient manner. than those in absence of the polymeric drug carrier.

The present invention further provides polymeric drug carriers which can deliver a bioactive agent into targeted cell.

Specifically the present invention provides a method for the intracellular delivery ofbio active agents comprising the steps of:
a) selecting at least one bioactive agents;
b) preparing a polymeric composition comprising an amphiphilic block copolymer comprised of a hydrophilic block and a hydrophobic block wherein said hydrophobic block has a terminal hydroxyl group that is substituted with a tocopherol or cholesterol group, a polylactic acid derivative having at least one carboxyl group at the end of the polymer;
c) mixing and dissolving said polymeric composition and said bioactive agents in a solvent and evaporating the solvent;
d) adding an aqueous solution to form a polymeric drug carrier with said bioactive agents entrapped therein in the solution; and
e) contacting said drug carrier with a cell to facilitate delivery of said bio active agents within said cell.

### Selecting at least one bioactive agents

The bioactive agents of the present invention can be any organic compound or any drug which exerts a desirable biological activity. This includes, but is not limited to, proteins, hormones such as testosterone, estradiol, estrogen, progesterone, triamcinolon acetate, dexamethasone, etc., genes, polypeptides, oligonuleotides, nucleotides, antibodies, drugs such as anticancer agents, anti-inflammatory agents, antifungal agents, antibiotics , anesthetics, antihypertensive agents, and agents for the treatment of diabetes, antihyperlipidemic agents, antiviral agents, agents for the treatment of Parkinson's disease, antidementia agents, antiemetics, immunosuppressants, antiulcerative agents, laxatives, antimalarial agents, and diagnostic imaging agents. Examples of anticancer drugs include paclitaxel, epirubicin, dactinomycin, bleomycin, mitomycin, docetaxel, 5-fluorouracil, methotrexate, camptothecin, etoposide, doxorubicin, dausorubicin, idarubicin, ara-C, cyclosporine A, etc., and derivatives thereof.

The above bioactive agent may be added to the polymeric composition in a weight-by-weight ratio of 0.1~20:80.0∼99.9 to be appropriately contained in the core of the micelles formed from the amphiphilic block copolymer and the polylactic acid derivative.

### Preparing a polymeric composition

The amphiphilic block copolymer of the present invention is preferably an A-B type diblock copolymer or B-A-B type triblock copolymer comprising a hydrophilic A block and a hydrophobic B block. The amphiphilic block copolymer, when placed in an aqueous phase, forms core-shell type polymeric micelles wherein the hydrophobic B block forms the core and the hydrophilic A block forms the shell. Preferably, the hydrophilic A block is a member selected from the group consisting of polyalkylene glycol, polyvinyl alcohol, polyvinyl pyrrolidone, polyacryl amide and derivatives thereof. More preferably, the hydrophilic A block is a member selected from the group consisting of monomethoxypolyethylene glycol, monoacetoxypolyethylene glycol, polyethylene glycol, polyethylene-co-propylene glycol, and polyvinyl pyrrolidone. Preferably, the hydrophilic A block has a number average molecular weight of 500 to 50,000 Daltons. More preferably, the hydrophilic A block has a number average molecular weight of 1,000 to 20,000 Daltons.

The hydrophobic B block of the amphiphilic block copolymer of the present invention is a highly biocompatible and biodegradable polymer selected from the group consisting of polyesters, polyanhydrides, polyamino acids, polyorthoesters and polyphosphazine. More preferably, the hydrophobic B block is one or more selected from the group consisting of polylactides, polyglycolides, polycaprolactone, polydioxan-2-one, polylactic-co-glycolide, polylactic-co-dioxan-2-one, polylactic-co-caprolactone, and polyglycolic-co-caprolactone. The terminal group of the hydrophobic block has a hydroxyl group, and the hydroxyl terminal group of the hydrophobic B block is substituted with a hydrophobic tocopherol or cholesterol group, both having excellent hydrophobicity, with the aim of increasing the hydrophobicity of the hydrophobic B block while maintaining its molecular weight. Tocopherol or cholesterol group is chemically bound to the hydroxyl terminal gourp of the hydrophobic B block using a linkage agent, e.g. a dicarboxylic acid such as succinic acid, malonic acid, glutaric acid, adipic acid. Tocopherol and cholesterol are biocompatible and hydrophobic compounds having a ring structure, which can increase the interior hydrophobicity of the polymeric micelles thereby enhancing the physical stability of the polymeric micelles. Preferably, the hydrophobic B block of the amphiphilic block copolymer has a number average molecular weight of 500 to 50,000 Daltons. More preferably, the hydrophobic B block of the amphiphilic block copolymer has a number average molecular weight 1,000 to 20,000 Daltons.

The ratio of the hydrophilic A block to the hydrophobic B block of the amphiphilic block copolymer of the present invention is preferably within the range of 3:7 to 8:2 by weight, and more preferably within the range of 4:6 to 7:3. If the content of the hydrophilic A block is too low, the polymer may not form polymeric micelles in an aqueous solution, and if the content is too high, the polymeric micelles formed are not stable.

In one embodiment, the amphiphilic block copolymer of the present invention may be represented by the following Formula:
R_{1'}-O-[R_{3'}]_{1'}-[R_{4'}]_{m'}-[R_{5'}]_{n'}-C(=O)-(CH₂)_{x'}-C(=O)-O-R_{2'} (I')
wherein R₁, is CH₃-, H-[R_{5'}]_{n'}-[R_{4'}]_{m'}-, or R_{2'}-O-C(=O)-(CH₂)_{x'}-C(=O)-[R_{5'}]ₙ-[R_{4'}]_{m'}-;
R_{2'} is tocopherol or cholesterol;
R₃, is -CH₂CH₂-O-, -CH(OH)-CH₂-, -CH(C(=O)-NH₂)-CH₂-, or 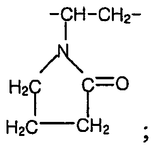
R_{4'} is -C(=O)-CHZ'-O-, wherein Z' is a hydrogen atom or methyl group;
R₅' is -C(=O)-CHY"-O-, wherein Y" is a hydrogen atom or methyl group, -C(=O)-CH₂CH₂CH₂CH₂CH₂-O-, or -C(=O)-CH₂OCH₂CH₂-O-;
1' is an integer from 4-1150;
m' is an integer from 1-300;
n' is an integer from 0-300; and
X' is an integer from 0-4.

The block copolymer having the hydrophobic block whose hydroxyl terminal group is substituted with tocopherol or cholesterol can be prepared according to the following methods. In one embodiment, a suitable linker, e.g. a dicarboxylic acid such as succinic acid, malonic acid, glutaric acid or adipic acid, is introduced into the hydroxyl group of tocopherol or cholesterol, and the carboxylated tocopherol or cholesterol is chemically bound to the hydroxyl terminal group of the hydrophobic B block.

In one embodiment, according to the method of US Patent No. 6,322,805, the amphiphilic block copolymer (mPEG-PLA) comprised of monomethoxypolyethylene glycol (mPEG; Mn=2,000) and polylactide (PLA; Mn=1,750) is weighed, and dehydrated using a vacuum pump at 120 °C, and then dissolved in acetonitrile or methylene chloride. Thereto is added tocopherol succinate or cholesterol succinate, and dicyclohexylcarbodiimide (DCC) and 4-dimethylaminopyridine (DMAP) are weighed and added thereto as an initiator and a catalyst, respectively, and the reaction is performed at room temperature. The reactant becomes opaque due to dicyclohexylurea (DCU) formed in the reaction between the terminal -OH of mPEG-PLA and -COOH of the hydrophobic compound. After 24 hours, DCU is removed by using a glass filter, and DMAP is extracted and removed with a hydrochloric acid aqueous solution. To this purified product solution is added MgSO₄ to remove any residual moisture, and then, precipitates are formed in a hexane/diethyl ether solvent in order to obtain the amphiphilic block copolymer to which tocopherol succinyl or cholesterol succinyl is bound, mPEG-PLA-tocopherol or mPEG-PLA-cholesterol (in which tocopherol or cholesterol is bound to PLA via succinic acid diester). The precipitated polymeric product is filtered, and then dried under vacuum to obtain the polymer as white particles.

In another embodiment, a carboxylated hydrophobic compound is activated with oxalyl chloride without any catalyst, and bound to the end of mPEG-PLA. That is, tocopherol (or cholesterol) succinate is reacted with oxalyl chloride, and then, excessive oxalyl chloride is removed under vacuum at room temperature. The mPEG-PLA is weighed and added thereto, and the reaction is performed at 100 °C for 12 hours to obtain mPEG-PLA-tocopherol (or cholesterol). The synthesized polymer is dissolved in acetonitrile or methylene chloride, precipitated in hexane/diethyl ether, and filtered.

In the above two preparation processes, tocopherol (or cholesterol) malonate, tocopherol (or cholesterol) glutarate, or tocopherol (or cholesterol) adipate, etc. can be used instead of tocopherol (or cholesterol) succinate.

In another embodiment, tocopherol or cholesterol is bound to the end of mPEG-PLA by using a dichloride compound as a linkage agent. Specifically, tocopherol or cholesterol is weighed and dehydrated by using a vacuum pump at 50 °C. Excessive linkage agent is added thereto, and the reaction is performed for 12 hours. After the reaction is completed, the excessively added linkage agent is removed under vacuum at 100 °C. Thereto is added weighed xnPEG-PLA, and the reaction is performed at 100 °C for 12 hours. The synthesized polymer is dissolved in methylene chloride, and precipitated in hexane/diethyl ether in order to obtain the amphiphilic block copolymer in which tocopherol or cholesterol is bound to PLA via succinic acid diester, i.e. mPEG-PLA-tocopherol or mPEG-PLA-cholesterol. The precipitated polymeric product is filtered, and dried under vacuum to obtain the polymer as white particles. The linkage agent which can be used in the reaction may be selected from such dichloride compounds as succinyl chloride, oxalyl chloride, malonyl chloride, glutaryl chloride, adipoyl chloride, etc.

One or more ends of the polylactic acid derivative of the present invention are covalently bound to at least one carboxylic acid or carboxylate salt. The other end of the polylactic acid derivative of the present invention may be covalently bound to a functional group selected from the group consisting of hydroxyl, acetoxy, benzoyloxy, decanoyloxy and palmitoyloxy groups. The carboxylic acid or carboxylate salts function as a hydrophilic group in an aqueous solution of pH 4 or higher which enables the polylactic acid derivative to form polymeric micelles therein. When the polylactic acid derivatives of the present invention are dissolved in an aqueous solution, the hydrophilic and hydrophobic components present in the polylactic acid derivative should be balanced in order to form polymeric micelles. Therefore, the number average molecular weight of the polylactic acid derivative of the present invention is preferably within the range of 500 to 2,500 Daltons. The molecular weight of the polylactic acid derivative can be adjusted by controlling the reaction temperature, time, and the like, during the preparation process.

The polylactic acid derivative is preferably represented by the following formula:

RO-CHZ-[A]ₙ-[B]ₘ-COOM (I)

wherein A is -COO-CHZ-; B is -COO-CHY , -COO-CH₂CH₂CH₂CH₂CH₂- or - COO-CH₂CH₂OCH₂; R is a hydrogen atom, acetyl, benzoyl, decanoyl, palmitoyl, methyl or ethyl group; Z and Y each are a hydrogen atom, methyl, or phenyl group; M is H, Na, K, or Li; n is an integer from 1 to 30, and m is an integer from 0 to 20.

One end of the polylactic acid derivative of the present invention is covalently bound to a carboxyl group or an alkali metal salt thereof, preferably, an alkali metal salt thereof. The metal ion in the alkali metal salt which forms the polylactic acid derivative is monovalent, e.g. sodium, potassium or lithium. The polylactic acid derivative in the metal ion salt form is a solid at room temperature, and is very stable because of its relatively neutral pH.

More preferably, the polylactic acid derivative is represented by the following formula:

RO-CHZ-[COO-CHX]ₚ-[COO-CHY']_{q}-COO-CHZ-COOM (II)

wherein X is a methyl group; Y' is a hydrogen atom or phenyl group; p is an integer from 0 to 25; q is an integer from 0 to 25, provided that p+q is an integer from 5 to 25; R, Z and M are the same as defined in Formula (I).

In addition, polylactic acid derivatives of the following formulas (III), (IV) and (V) are also suitable for the following formula:

RO-PAD-COO-W'-M' (III)

wherein W-M' is 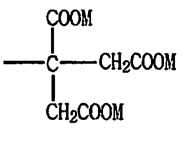 or 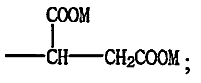 ; the PAD is a member selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-Lactic acid and caprolactone, and a copolymer of D,L-lactic acid and 1,4-dioxan-2-one; R and M are the same as defined in Formula (I).

S-O-PAD-COO-Q (IV)

wherein S is 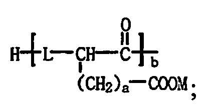 ; L is -NR₁- or -O-; R₁ is a hydrogen atom or C₁₋₁₀alkyl; Q is CH₃, CH₂CH₃, CH₂CH₂CH₃, CH₂CH₂CH₂CH₃, or CH₂C₆H₅; a is an integer from 0 to 4; b is an integer from 1 to 10; R and M are the same as defined in Formula (I); and PAD is the same as defined in Formula (III). 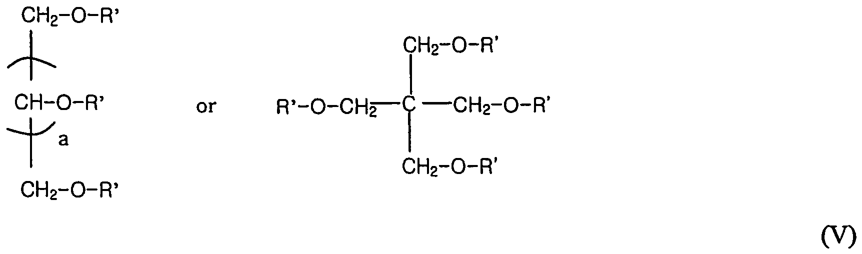 wherein R' is -PAD-O-C(O)-CH₂CH₂-C(O)-OM and M is the same as defined in formula (I); PAD is the same as defined in Formula (III); a is an integer from 1 to 4 (when a=1, it is a 3-arm PLA-COONa; if a=2, it is a 4-arm PLA-COONa; when a=3, it is a 5-arm PLA-COONa, and if a=4, it is 6-arm PLA-COONa).

The initiator for the synthesis of the polymers (Formula V) includes glycerol, erythritol, threltol, pentaerytritol, xylitol, adonitol, sorbitol and mannitol.

The polymeric composition of the present invention may contain 0.1 to 99.9 wt% of the amphiphilic block copolymer and 0.1 to 99.9 wt% of the polylactic acid derivative based on the total weight of the amphiphilic block copolymer and the polylactic acid derivative. Preferably, the polymeric composition of the present invention contains 20 to 95 wt% of the amphiphilic block copolymer and 5 to 80 wt% of the polylactic acid derivative. More preferably, the polymeric composition of the present invention contains 50 to 90 wt% of the amphiphilic block copolymer and 10 to 50 wt% of the polylactic acid derivative.

Although the polylactic acid derivatives of the present invention alone can form micelles in an aqueous solution with a pH 4 or higher, the polymeric compositions comprising amphiphilic block copolymer and polylactic acid derivatives can form micelles in an aqueous solution irrespective of the pH of the solution, and the polymeric compositions of the present invention may be used at a pH within the range of 1 to 10, preferably at a pH within the range of 4 to 8. The particle size of the micelles or nanoparticles prepared from the polymeric compositions of the present invention may be adjusted to be within the range of 1 to 400 nm, and preferably from 5 to 200 nm, depending on the molecular weight of the polymers and the ratio of the polylactic acid derivative to the amphiphilic block copolymer.

In one embodiment of the present invention, the carboxyl terminal group of the polylactic acid derivative is bound or fixed with a di- or tri-valent metal ion. The metal ion-fixed polymeric composition can be prepared by adding the di- or tri-valent metal ion to the polymeric composition of the amphiphilic block copolymer and the polylactic acid derivative. The polymeric micelles or nanoparticles may be formed by changing the amount of the di- or tri-valent metal ion added for binding or fixing the carboxyl terminal group of the polylactic acid derivative.

The di- or tri-valent metal ion is preferably a member selected from the group consisting of Ca²⁺, Mg²⁺, B²⁺ Cr³⁺, Fe³⁺, Mn²⁺ Ni²⁺, Cu²⁺ Zn²⁺, and Al³⁺ The di- or tri-valent metal ion may be added to the polymeric composition of the amphiphilic block copolymer and the polylactic acid derivative in the form of a sulfate, chloride, carbonate, phosphate or hydroxylate, and preferably, in the form of CaCl₂, MgCl₂, ZnCl₂, AlCl₃, FeCl₃, CaCO₃, MgCO₃, Ca₃(PO₄)₂, Mg₃(PO₄)₂, AlPO₄, MgSO₄, Ca(OH)₂, Mg(OH)₂, Al(OH)₃, or Zn(OH)₂.

Either polymeric micelles or nanoparticles can be prepared by changing the number of equivalents of the metal ion added. Specifically, if a divalent metal ion is added at 0.5 equivalents or less with respect to the carboxyl terminal groups, the metal ion that can form bonds with the carboxyl terminal group of the polylactic acid derivative is insufficient; and thus, polymeric micelles are formed. If a divalent metal ion is added at 0.5 equivalents or more, the metal ion that can form bonds with the carboxyl terminal group of the polylactic acid derivative is sufficient to firmly fix the micelles; and thus, nanoparticles are formed.

In addition, the drug release rate from the polymeric micelles or nanoparticles may be adjusted by changing the number of equivalents of the metal ion added. If the metal ion is present at 1 equivalent or less with respect to that of the carboxyl group of the polylactic acid derivative, the number available to bond to the carboxyl terminal group of the polylactic acid derivative is decreased, and so the drug release rate is increased. If the metal ion is present at 1 equivalent or more, the number available to bond to the carboxyl terminal group of the polylactic acid derivative is increased, and so the drug release rate is decreased. Therefore, to increase the drug release rate in the blood, the metal ion is used in a small equivalent amount, and to decrease the drug release rate, the metal ion is used in a large equivalent amount.

The metal ion-fixed polymeric compositions of the present invention may contain 5 to 95wt% of the amphiphilic block copolymer, 5 to 95wt% of the polylactic acid derivative and 0.01 to 10 equivalents of the di- or tri-valent metal ion with respect to the number of equivalents of the carboxyl terminal groups of the polylactic acid derivatives. Preferably, they contain 20 to 80wt% of the amphiphilic block copolymer, 20 to 80wt% of the polylactic acid derivative and 0.1 to 5 equivalents of the di- or tri-valent metal ion, and more preferably, 20 to 60wt% of the amphiphilic block copolymer, 40 to 80wt% of the polylactic acid derivative and 0.2 to 2 equivalents of the di- or tri-valent metal ion.

### Mixing and dissolving the polymeric composition and the bioactive agents in a solvent and evaporating the solvent; and formulation of a solution of a drug carrier with a bioactive agent entrapped therein

The drug carrier of the present invention can be a polymeric micelle or a nanoparticle formed from polymeric compositions comprising an amphiphilic block copolymer which is comprised of a hydrophilic block and a hydrophobic block wherein said hydrophobic block has a terminal hydroxyl group that is substituted with a tocopherol or cholesterol group, and a polylactic acid derivative having at least one terminal carboxyl group at the end of the polymer.

The nanoparticles or micelles of the present invention can be formed from polymeric compositions of an amphiphilic block copolymer comprised of a hydrophilic block and a hydrophobic block, wherein the hydrophobic block has a hydroxyl terminal group which is substituted with a tocopherol or cholesterol group, a polylactic acid derivative having at least one terminal carboxyl group at the end which is bound or fixed with a di- or tri-valent metal ion.

The bioactive agents can be entrapped in the micelles or nanoparticles or they can be incorporated within the micelles or nanoparticles of the present invention by formation of a stable ionic complex with the carboxyl group of the biodegradable polylactic acid derivative according to the bioactive agents.

The amphiphilic block copolymer, the polylactic acid derivative, and the poorly water-soluble drug at certain ratios can be dissolved in one or more mixed organic solvents selected from the group consisting of acetone, ethanol, methanol, ethyl acetate, acetonitrile, methylene chloride, chloroform, acetic acid and dioxane. The organic solvent can be removed therefrom to prepare a homogenous mixture of the poorly water-soluble drug and the polymer. The homogenous mixture of the poorly water-soluble drug and the polymeric composition of the present invention can be added to an aqueous solution with a pH of 4 to 8, at 0 to 80 °C, resulting in poorly water-soluble drug-containing mixed polymeric micelle aqueous solution. The above drug-containing polymeric micelle aqueous solution can then be lyophilized to prepare the polymeric micelle composition in the form of a solid.

An aqueous solution containing 0.001 to 2 M of the di- or tri-valent metal ion is added to the poorly water-soluble drug-containing mixed polymeric micelle aqueous solution. The mixture is slowly stirred at room temperature for 0.1 to 1 hour and then lyophilized to prepare the metal ion-fixed polymeric micelle or nanoparticle composition in the form of a solid.

### Contacting the drug carrier with a cell

For oral or parenteral administration of a bioactive agent, the bioactive agent is entrapped in the drug carrier and is thereby solubilized. Particularly, the metal ion-fixed polymeric micelles or nanoparticles are retained in the bloodstream for a long period of time and accumulate in the target lesions. The bioactive agent is released from the hydrophobic core of the micelles to exert a pharmacological effect while the micelles are degraded.

For parenteral delivery, the polymeric composition may be administered intravenously, intramuscularly, intraperitoneally, transnasally, intrarectally, intraocularly, or intrapulmonarily. For oral delivery, the bioactive agent is mixed with the drug carrier of the present invention, and then administered in the form of a tablet, capsule, or aqueous solution. The polymeric composition of the invention may be administered with various ranges according to the requirements of the particular drug. As is well known in the medical arts, dosages for any one patient depends upon many factors, including the patient's weight, body surface area, age, the particular compound to be administered, sex, time and route of administration general health, and other drugs being administered concurrently.

### Cellular internalization of a bioactive agent

Cellular internalization of a bioactive agent can be studied with flow cytometry and confocal microscopy using drug-containing compositions. As an example of the present invention, human uterine cancer cell lines(MES-SA; MES-SA/Dx-5) and human breast cancer cell lines(MCF-7; MCF-7/ADR) were treated with the drug compositions of the present invention. Cell lines MES-SA and MCF-7 are doxorubicin-sensitive and cell lines MES-SA/Dx-5; MCF-7/ADR are doxorubicin-resistant.

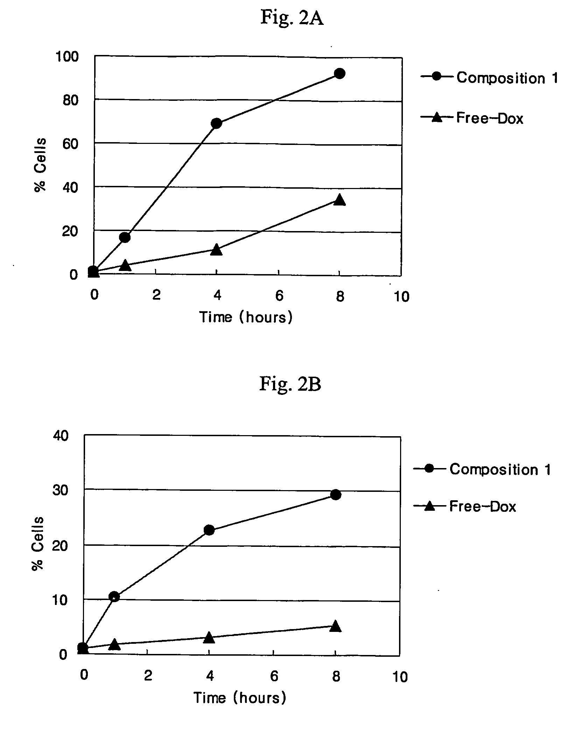

As shown in Figs. 2A to 3B and Tables 2A and 2B, the drug enters more effectively into the cells from the drug composition of the present invention (Composition 1) than from the conventional solution formulation (Free-Dox). Most notably, the number of cells which absorbed the drug was five times higher from the drug composition of the present invention than from the conventional solution formulation. Particularly the uptake of doxorubicin into cells is remarkable in the drug resistant cell line. Thus, there is a good possibility that the drug composition of the present invention can be used to overcome multi-drug resistance in chemotherapy.

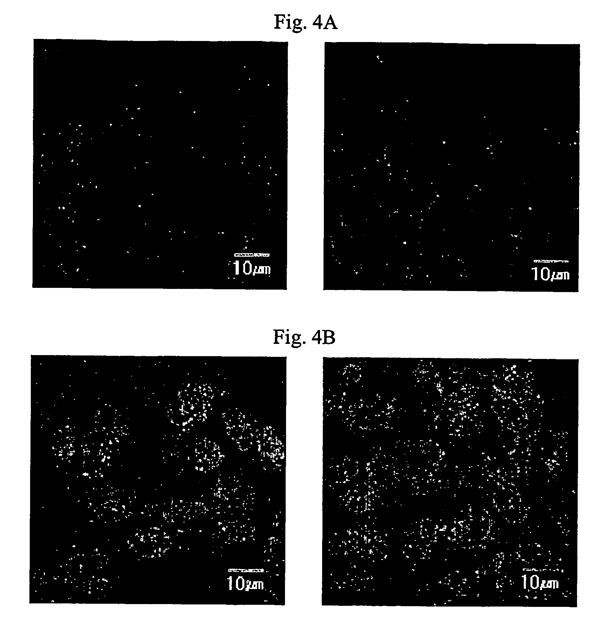

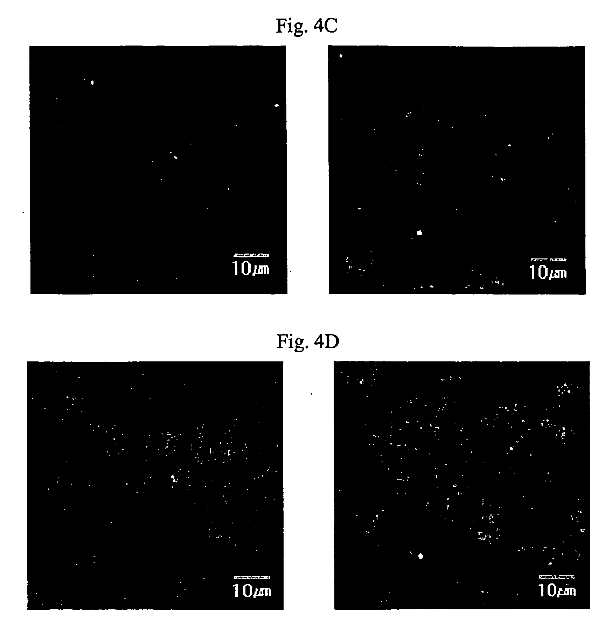

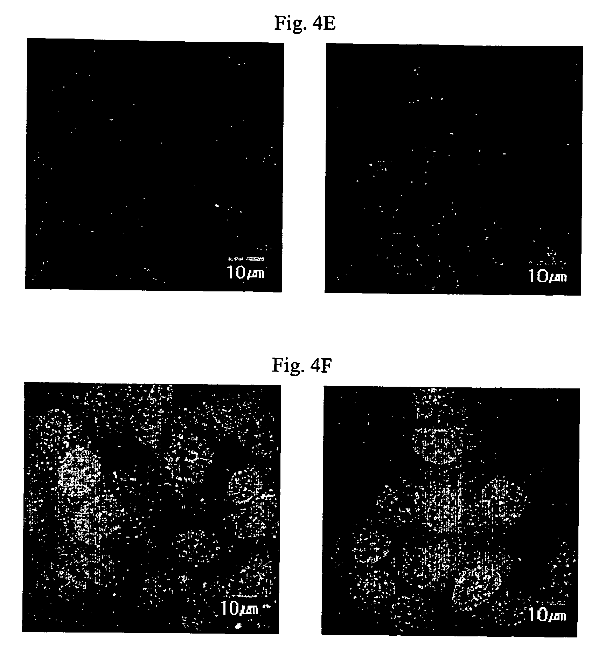

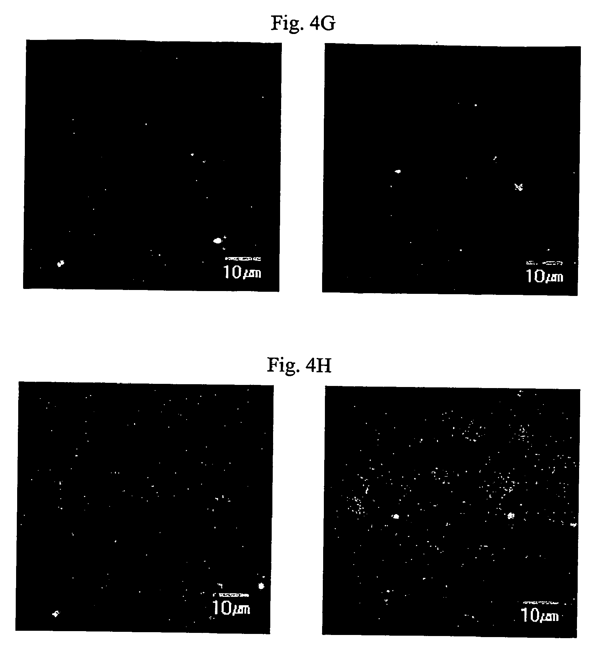

The confocal images in Figs. 4A to 4H visualize the flow cytometry results: much higher amounts of drug were absorbed by the cells when the drug composition of the present invention was treated. In Figs. 4A to 4H, the left side pictures are the confocal images after treatment with the conventional solution formulation and the images on the right side are after treatment with the compositions of the present invention. And as shown in Figs. 4B, 4D, 4F, and 4H, the micelles or nanoparticles were detected in the cytoplasmic and nuclear compartments.

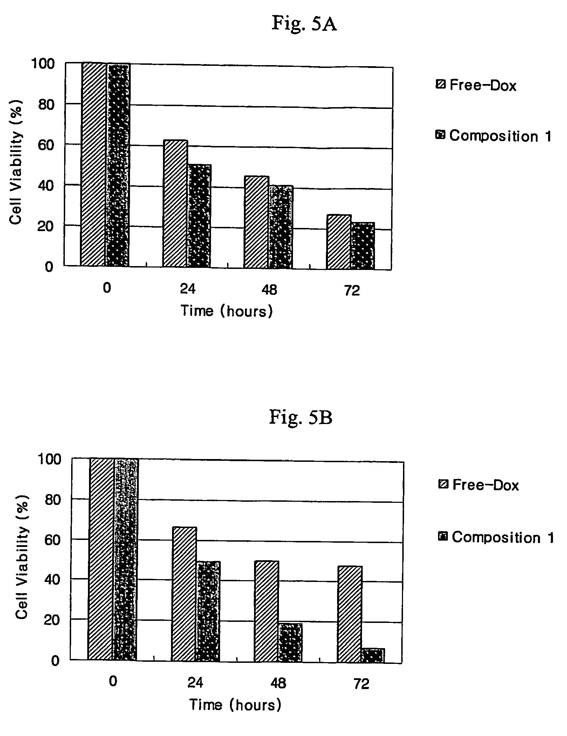

Furthermore, the MTT assay results shown in Figs. 5A to 5B and Table 3 support the results of the flow cytometry and confocal microscopy studies. For the MTT assay, a doxorubicin-containing composition of the present invention (Composition 1) and a conventional doxorubicin formulation (Free-Dox) were tested on the human uterine cancer cell lines MES-SA (doxorubicin-sensitive cell line) and MES-SA/Dx-5 (doxorubicin-resistant cell line). The cytotoxic activity on the doxorubicin-sensitive cells was similar in both compositions as shown in Figs. 5A, but the drug composition of the present invention showed 6.7 times higher activity at three days after treatment than the conventional solution formulation when treating the doxorubicin-resistant cells as shown in Fig. 5B. This difference in activity is due to the characteristics of the drug-resistant cell lines in which the P-glycoproteins (P-gp) are overexpressed and they continuously extrude the cytotoxic drugs from the cell. Since free drug cannot be concentrated within the drug-resistant cells, this result implies that the drug carrier of the present invention enters together the cell with the drug incorporated in the drug carrier.

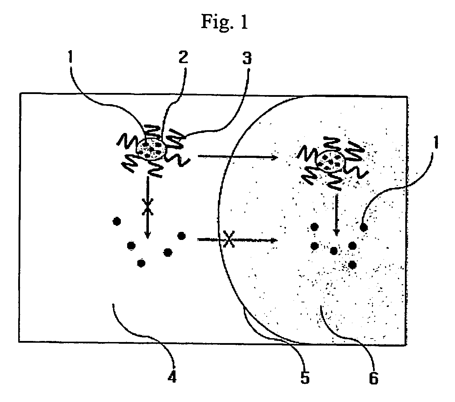

From the physical stability of the drug carrier of the present invention and the results of the cellular internalization study, it can be concluded that most of the bioactive agent-containing compositions of the present invention enter the cell cytoplasm in the form of a micelle or nanoparticle by endocytosis without collapse of its structure. This cellular internalization process is schematically shown in Fig 1: 1 represents a drug; 2 represents the inner core of a micelle or nanoparticle; 3 represents the outer shell of a micelle or nanoparticle; 4 represents the extracellular fluid; 5 represents the cell membrane; and represents the cytoplasm.

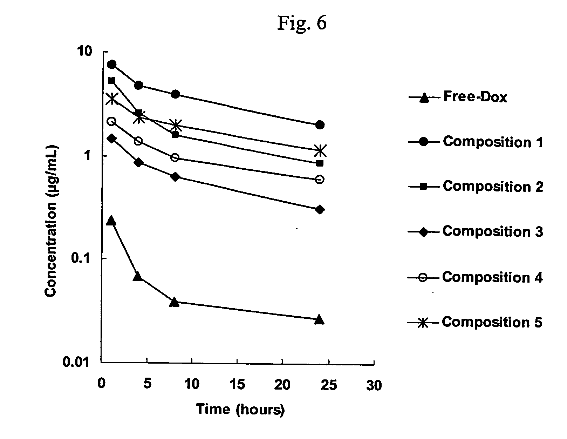

A pharmacokinetic experiment was performed with Sprague-Dawley rats (200~250g) using doxorubicin-containing compositions of the present invention (Compositions 1 to 5) and the conventional doxorubicin formulation (Free-Dox). As shown in Fig. 6 and Table 4, the compositions of the present invention exhibited prolonged blood circulation time compared to the conventional doxorubicin formulation. The bioavailability calculated from the area under the blood concentration-time curve (AUC) for the composition 1 of the present invention was 63 times higher than that of conventional doxorubicin formulation.

The drug carriers of the present invention can provide for a prolonged systemic circulation time due to their small size (<100nm), their hydrophilic shell which minimizes uptake by the MPS, and their high molecular weight which prevents renal excretion. The drug carriers of the present invention can be used as carriers for water-soluble drugs, peptides and proteins as well as for poorly water-soluble drugs. As illustrated by the cellular internalization study, the bioactive agent-containing compositions of the present invention form such stable micelles or nanoparticles in aqueous media that they enter the cytoplasm in the form of micelles or nanoparticles by endocytosis without collapse of their structure. Furthermore, higher accumulation of a bioactive agent in tumor tissue can be achieved with the composition of the present invention.

### BEST MODE TO CARRY OUT THE INVENTION

### Preparation Example 1

### Amphiphilic block copolymer of PEG and PLA (mPEG-PLA)

A mixture of 20g of monomethoxy polyethyleneglycol (mPEG with a molecular weight of 2,000), 20g of D,L-lactide which was recrystallized from ethyl acetate, and 0.2g of stannous octoate which was dissolved in 5ml toluene were added to a reactor equipped with a mechanical stirrer and a distillation set. Residual toluene was evaporated at 120 °C . The reaction was carried out under vacuum (25mmHg). After 6 hours of polymerization occurring, the resulting polymer was dissolved in dichloromethane and poured into cold diethyl ether (4°C) to precipitate the polymer. The precipitated polymer was washed twice with diethyl ether and dried under vacuum (0.1mmHg) for 24 hours. The molecular weight of the block copolymer determined by nuclear magnetic resonance (NMR) spectroscopy was 2,000-1,800 (2,000 for PEG block and 1,800 for PLA block).

### Preparation Example 2

### Amphiphilic block copolymer of PEG and PLGA (mPEG-PLGA)

A diblock copolymer (mPEG-PLGA, LA:GA=70:30 by weight) was prepared by the same procedure described in Preparation Example 1, using 14g of D,L-lactide and 6g of glycolide instead of 20g of D,L-lactide. The molecular weight of the block copolymer determined by NMR was 2,000-1,750 (2,000 for the PEG block and 1,750 for the PLGA block).

### Preparation Example 3

### Amphiphilic block copolymer of PEG and PCL (mPEG-PCL)

A diblock copolymer (mPEG-PCL) was prepared by the same procedure described in Preparation Example 1, using 20g of ε -caprolactone instead of D,L-lactide. The molecular weight of the block copolymer determined by NMR was 2,000-1,800 (2,000 for the PEG block and 1,800 for the PCL block).

### Preparation Example 4

### Amphiphilic block copolymer of PEO and PLA (PLA-PEO-PLA)

A triblock copolymer (PLA-PEO-PLA) was prepared by the same procedure described in Preparation Example 1, using 20g of polyethyleneglycol (PEG with a molecular weight of 2,000) instead of monomethoxy polyethyleneglycol (mPEG with a molecular weight of 2,000). The molecular weight of the block copolymer determined by NMR was 850-2,000-850 (2,000 for the PEO block and 900 for each PLA block).

### Preparation Example 5

### Tocopherol succinate

A mixture of 8.6g of tocopherol, 2.4g of succinic anhydride and 2.9g of 4-(dimethylamino) pyridine (DMAP) were dissolved in 100 ml of 1,4-dioxane in a reactor equipped with a mechanical stirrer. The reaction was carried out at room temperature. After 24 hours of stirring, the reaction mixture was introduced into an HCl solution to precipitate the tocopherol succinate (10.2 g; yield = 96%).

### Preparation Example 6

### Cholesteryl succinate

Cholesteryl succinate was prepared (9.1 g; yield = 94%) by the same procedure described in Preparation Example 5, using 7.7g of cholesterol instead of tocopherol.

### Preparation Example 7

### Amphiphilic block copolymer having a tocopherol group at the end of the hydrophobic block (mPEGPLA-Toco)

A mixture of 10.0 g (2.6mmole) of mPEG-PLA prepared from Preparation Example 1 and 1.7 g (3.2mmole) of tocopherol succinate prepared from Preparation Example 5 were dissolved in 50 ml of acetonitrile in a reactor equipped with a mechanical stirrer. 0.78g (3.8mmole) of dicyclohexylcarbodiimide (DCC) and 0.046 g (0.38mmole) of 4-(dimethylamino)pyridine (DMAP) were used as catalysts. After stirring for 24 hours at room temperature, the mixture was filtered using a glass filter to remove dicyclohexylcarbourea. The remaining catalysts were extracted out with an aqueous HCl solution, and magnesium sulfate was added into the polymer solution to remove water. The resulting product (mPEG-PLA-Toco) was recrytallized from a cosolvent of n-hexane/diethyl ether (6/4, v/v). The recrytallized product was filtered and dried under vacuum to give a white powdered product (9.9 g; yield = 87%).

### Preparation Example 8

### Amphiphilic block copolymer having a tocopherol group at the end of the hydrophobic block (mPEGPLGA-Toco)

An amphiphilic block copolymer (mPEG-PLGA-Toco) was prepared (9.5 g; yield = 83%) by the same procedure described in Preparation Example 7, using 9.8g (2.6mmole) of mPEG-PLGA prepared from preparation example 2 instead of mPEG-PLA prepared from Preparation Example 1.

### Preparation Example 9

### Amphiphilic block copolymer having a tocopherol group at the end of the hydrophobic block (mPEG-PCL-Toco)

An amphiphilic block copolymer (mPEG-PCL-Toco) was prepared (10.1 g; yield = 89%) by the same procedure described in Preparation example 7, using 10.0g (2.6mmole) of mPEG-PCL prepared from preparation example 3 instead of mPEG-PLA prepared from Preparation Example 1.

### Preparation Example 10

### Amphiphilic block copolymer having a tocopherol group at the end of the hydrophobic block (Toco-PLA-PEO-PLA-Toco)

An amphiphilic block copolymer (Toco-PLA-PEO-PLA-Toco) was prepared (9.2 g; yield = 84%) by the same procedure described in Preparation Example 7, using 9.6g (2.6mmole) of PLA-PEO-PLA prepared from Preparation Example 4 instead of mPEG-PLA prepared from Preparation Example 1.

### Preparation Example 11

### Amphiphilic block copolymer having a cholesterol group at the end of the hydrophobic block (mPEG-PLA-Chol)

An amphiphilic block copolymer (mPEG-PLA-Chol) was prepared (9.7 g; yield = 86%) by the same procedure described in Preparation Example 7, using 1.6 g (3.2mmole) of cholesteryl succinate) prepared from Preparation Example 6 instead of tocopherol succinate prepared from Preparation Example 5.

### Preparation Example 12

### Biodegradable polyester (PLMA-COONa)

### (1) Preparation of PLMA-COOH

A mixture of 7.5g of D,L-lactic acid (0.083mole) and 2.5g of D,L-mandelic acid (0.016mole) were added to a reactor equipped with a mechanical stirrer and a distillation set. Moisture was evaporated at 80°C for 1 hour under reduced pressure (25mmHg) with an aspirator. The reaction was carried out at an elevated temperature of 180 °C for 5 hours under vacuum (10mmHg). The resulting product was added to distilled water, the precipitated polymer was further washed with distilled water. The polymer product was then added to 0.1 liter of distilled water, and the pH of the aqueous solution was adjusted between 6 and 8 by the addition of sodium hydrogen carbonate portionwise thereto dissolving the polymer. The water-insoluble polymer was separated and removed by centrifugation or filtration. A 1 N hydrochloric acid solution was added dropwise thereto and the polymer was precipitated in the aqueous solution. The precipitated polymer was washed twice with distilled water, isolated and dried under reduced pressure to obtain a polymer having a carboxyl end group (6.7 g of PLMA-COOH, yield = 67%). The number average molecular weight of the polymer determined by NMR was 1,100.

### (2) Preparation of PLMA-COONa

A solution of 5g of PLMA-COOH polymer was dissolved in acetone in a reactor equipped with a mechanical stirrer and a distillation set. The solution was stirred slowly at room temperature, and sodium hydrogen carbonate solution (1 N) was slowly added thereto to reach a pH of 7. Anhydrous magnesium sulfate was added thereto to remove any residual moisture. The mixture was filtered, remaining acetone was evaporated with a rotary evaporator, and a white solid product was obtained therefrom. The solid product was dissolved again in anhydrous acetone, the solution was filtered to remove insoluble particles, the acetone was evaporated off to give the final product, PLMA-COONa, as a white solid (yield: 95%).

### Preparation Example 13

### Biodegradable polyester (3arm-PLA-COOK)

### (1) Preparation of 3arm-PLA-OH

1.0g (0.011mole) of glycerol was added to a reactor equipped with a mechanical stirrer and a distillation set. Moisture was evaporated at 80 °C for 30 minutes. 0.036g (0.089mmole) of stannous octoate in toluene was added thereto, the residual toluene was evaporated at 120 °C, and 36g (0.25mole) of D,L-lactide which was recrystallized from ethyl acetate was introduced into the reactor. The reaction was carried out at 130°C under vacuum (20mmHg). After 6 hours of polymerization, the resulting polymer was dissolved in acetone and an aqueous NaHCO₃ solution (0.2 N) was added dropwise thereto to precipitate the polymer. The precipitated polymer was washed three times with distilled water and dried under reduced pressure to give a white powder form of the polymer (3arm-PLA-OH). The molecular weight of the polymer determined by NMR spectroscopy was 3,050.

### (2) Preparation of 3arm-PLA-COOH

10g (3.28mmole) of 3arm-PLA-OH polymer prepared above was added to a reactor equipped with a mechanical stirrer and a distillation set. Moisture was evaporated at 120 °C for 1 hour. 1.96g (19.6mmole) of succinic anhydride was added thereto, and the reaction was carried out at 125 °C for 6 hours. The resulting polymer was dissolved in acetone and distilled water was added dropwise thereto to precipitate the polymer. The precipitated polymer was dissolved in an aqueous NaHCO₃ solution (0.2 N) at 60 °C, and aqueous HCl solution (1N) was added dropwise thereto to precipitate the polymer. The precipitated polymer was washed three times with distilled water and dried under vacuum to give a white powder form of the polymer (3arm-PLA-COOH). The molecular weight of the polymer determined by NMR spectroscopy was 3,200.

### (3) Preparation of 3arm-PLA-COOK

Finally, a biodegradable polyester (3arm-PLA-COOK) was prepared (yield = 90%) by the same procedure described in Preparation Example 12, using 5g of the 3arm-PLA-COOH polymer prepared above and a potassium hydrogen carbonate solution(1 N) instead of the PLMA-COOH polymer and sodium hydrogen carbonate solution(1 N).

### Preparation Example 14

### Biodegradable polyester (4arm-PLA-COONa)

### (1) Preparation of 4arm-PLA-OH

A 4arm-PLA-OH polymer was prepared by the same procedure described in Preparation Example 13, using 1.5g (0.011mole) of pentaerythritol instead of glycerol. The molecular weight of the polymer (4arm-PLA-OH) determined by NMR spectroscopy was 3,100.

### (2) Preparation of 4arm-PLA-COOH

A 4arm-PLA-COOH polymer was prepared by the same procedure described in Preparation Example 13, using 10g(3.23 mmole) of the 4arm-PLA-OH polymer prepared above and 2.58g (25.8mmole) of succinic anhydride instead of 10g (3.28mmole) of the 3arm-PLA-OH polymer and 1.96g (19.6mmole) of succinic anhydride. The molecular weight of the polymer (4arm-PLA-COOH) determined by NMR spectroscopy was 3,300.

### (3) Preparation of 4arm-PLA-COONa

Finally, a biodegradable polyester (4arm-PLA-COONa) was prepared (yield = 92%) by the same procedure described in Preparation Example 12, using 5g of the 4arm-PLA-COOH polymer prepared above instead of the PLMA-COOH polymer.

### Preparation Example 15

### Biodegradable polyester (PLA-COONa)

### (1) Preparation of PLA-COOH

A PLA-COOH was prepared (yield = 78%) by the same procedure described in Preparation Example 12, using 10g of D,L-lactic acid (0.11mmole). The number average molecular weight of the polymer determined by NMR was 1,100.

### (2) Preparation of PLA-COONa

A biodegradable polyester (PLA-COONa) was prepared (yield = 92%) by the same procedure described in Preparation Example 12, using 5g of the PLA-COOH polymer prepared above instead of the PLMA-COOH polymer.

### Example 1

### Drug-containing composition

### (Composition 1: Doxorubicin / mPEGPLA-Toco / PLMA-COONa)

10mg of doxorubicin hydrochloride was dissolved in 5ml of ethanol-water (9:1 v/v) in a round-bottomed flask. 810mg of the amphiphilic block copolymer prepared from preparation example 7 (mPEG-PLA-Toco) and 180mg of the biodegradable polyester prepared from Preparation Example 12 (PLMA-COONa) are added thereto and completely dissolved giving clear solution. The solvent was evaporated at an elevated temperature (60 °C) under vacuum with a rotary evaporator. A 3ml aqueous solution of lactose (20% by weight) was added and the flask was rotated at 100 rpm at 60 °C with a rotary evaporator to form micelles or nanoparticles in the aqueous medium. The solution was filtered using 0.22 µm PVDF membrane filter. The filtered solution was freeze-dried and stored in a refrigerator until use. Particle size of the micelles or nanoparticles in the filtered solution was measured by a dynamic light scattering method (DLS, ZetaPlus, Brookhaven Instruments Ltd.). The loading efficiency (wt.% of drug incorporated in the micelles or nanoparticles with respect to the initial drug used) was calculated from the doxorubicin content analyzed by HPLC using daunorubicin as the internal standard. The conditions for HPLC assay were as follows:
Injection volume: 75 µℓ
Flow rate: 1.0ml/min
Mobile phase: gradient increase of Solvent B from 15% to 85% for 40 minutes (Solvent A: 1% acetic acid; Solvent B: acetonitrile)
Temperature: Room Temperature
Column: C-18 (Vydac, multi-ring, pore size : 5 µm)
Wavelength; 485nm
And the particle size was measured according to a Dynamic Light Scattering (DLS) Method.

The results are summarized in Table 1.

### Example 2

### Drug-containing composition

### (Composition 2: Doxorubicin / mPEG-PLGA-Toco / PLMA-COONa)

A drug-containing composition (composition 2) was prepared by the same procedure described in Example 1, using mPEG-PLGA-Toco prepared from Preparation Example 8 instead of mPEG-PLA-Toco.

The results are summarized in Table 1.

### Example 3

### Drug-containing composition

### (Composition 3: Doxorubicin / mPEGPCL-Toco / 3arm-PLA-COOK)

A drug-containing composition (composition 3) was prepared by the same procedure described in Example 1, using mPEG-PCL-Toco prepared from Preparation Example 9 and 3arm-PLA-COOK prepared from Preparation Example 13 instead of mPEG-PLA-Toco and PLMA-COONa.

The results are summarized in Table 1.

### Example 4

### Drug-containing composition

### (Composition 4: Doxorubicin / Toco-PLA-PEO-PLA-Toco / 4arm-PLA-COONa)

A drug-containing composition (composition 4) was prepared by the same procedure described in Example 1, using Toco-PLA-PEO-PLA-Toco prepared from preparation example 10 and 4arm-PLA-COONa prepared from Preparation Example 14 instead of mPEG-PLA-Toco and PLMA-COONa.

The results are summarized in Table 1.

### Example 5

### Drug-containing composition

### (Composition 5: Doxorubicin / mPEGPLA-Chol / PLMA-COONa)

A drug-containing composition (composition 5) was prepared by the same procedure described in Example 1, using mPEG-PLA-Chol prepared from preparation Example 11 instead ofmPEG-PLA-Toco.

### Example 6

### Drug-containing composition

### (Composition 6: Epirubicin / mPEG-PLA-Toco / PLMA-COONa)

A drug-containing composition (composition 6) was prepared by the same procedure described in Example 1, using epirubicin instead of doxorubicin.

The results are summarized in Table 1.

### Example 7

### Drug-containing composition

### (Composition 7: Ca²⁺-fixed Paclitaxel / mPEGPLA-Toco / PLA-COONa)

### (1) Preparation of paclitaxel-containing aqueous solution

A mixture of 248.1 mg PLA-COONa prepared from Preparation Example 15, 7.5 mg of paclitaxel, and 744.3 mg of mPEG-PLA-Toco prepared from Preparation Example 7 were completely dissolved in 5 ml of ethanol to obtain a clear solution. Ethanol was removed therefrom to prepare a paclitaxel-containing polymeric composition. Distilled water(6.2 ml) was added thereto and the mixture was stirred for 30 minutes at 60 °C to prepare the paclitaxel-containing aqueous solution.

### (2) Fixation with the divalent metal ion

0.121 ml (0.109 mmol) of a 0.9 M aqueous solution of anhydrous calcium chloride was added to the paclitaxel-containing aqueous solution prepared above, and the mixture was stirred for 20 minutes at room temperature. The mixture was filtered using 0.22 µm PVDF membrane filter. The filtered solution was freeze-dried and stored in a refrigerator until use.

The results are summarized in Table 1.

### Example 8

### Drug-containing composition

### (Composition 8: Mg²⁺-fixed Paclitaxel / mPEGPLGA-Toco / PLMA-COONa)

A Mg²⁺-fixed paclitaxel-containing composition was prepared by the same procedure described in Example 7 except that 248.1mg of PLMA-COONa (Mn: 1,096) of Preparation Example 12, 7.5 mg of paclitaxel, 744.3 mg of mPEG-PLGA-Toco of preparation Example 8 and 0.230 ml (0.113 mmol) of a 0.5M aqueous solution of magnesium chloride 6 hydrate (Mw:203.31) were used.

The results are summarized in Table 1.

### Example 9

### Drug-containing composition

### (Composition 9: Ca²⁺-fixed Paclitaxel / mPEGPLA-Toco / PLMA-COONa)

A Cg²⁺-fixed paclitaxel-containing composition was prepared by the same procedure described in Example 7 except that 248.1mg of PLMA-COONa of Preparation Example 12, 7.5 mg of paclitaxel, 744.4 mg of mPEG-PLA-Toco of Preparation Example 7 and 0.230 ml (0.113 mmol) of a 0.9 M aqueous solution of anhydrous calcium chloride were used.

The results are summarized in Table 1.

### Example 10

### Drug-containing composition

### (Composition 10: Ca²⁺-fixed Paclitaxel / mPEGPLA-Chol / PLMA-COONa)

A Ca²⁺-fixed paclitaxel-containing composition was prepared by the same procedure described in Example 7 except that 248.1mg of PLMA-COONa of Preparation Example 12, 7.5 mg of paclitaxel, 744.4 mg of mPEG-PLA-Chol of Preparation Example 11 and 0.230 ml (0.113 mmol) of a 0.9 M aqueous solution of anhydrous calcium chloride were used.

The results are summarized in Table 1.

**Table 1.**

| | Drug | Amphiphilic block copolymer | Biodegradable polyester | Entrapment Efficiency (%) | particle size (mn) |
|---|---|---|---|---|---|
| Initial wt. | 10 mg | 810 mg | 180 mg | - | - |
| Comp. 1 | Doxorubicin | mPEG-PLA-Toco | PLMA-COONa | 98 | 32 |
| Comp. 2 | Doxorubicin | mPEG-PLGA-Toco | PLMA-COONa | 97 | 28 |
| Comp. 3 | Doxorubicin | mPEG-PCL-Toco | 3arm-PLA-COOK | 95 | 41 |
| Comp. 4 | Doxorubicin | Toco-PLA-PEO-PLA-Toco | 4arm-PLA-COONa | 95 | 57 |
| Comp. 5 | Doxorubicin | mPEG-PLA-Chol | PLMA-COONa | 96 | 35 |
| Comp. 6 | Epirubicin | mPEG-PLA-Toco | PLMA-COONa | 97 | 38 |
| Comp. 7 | Paclitaxel | mPEG-PLA-Toco | PLA-COONa | 99 | 29 |
| Comp. 8 | Paclitaxel | mPEG-PLGA-Toco | PLMA-COONa | 101 | 30 |
| Comp. 9 | Paclitaxel | mPEG-PLA-Toco | PLMA-COONa | 100 | 34 |
| Comp. 10 | Paclitaxel | mPEG-PLA-Chol | PLMA-COONa | 99 | 34 |

### Example 11

### Evaluation of the intracellular uptake of a drug: Flow cytometry

To evaluate the intracellular uptake of a bioactive agent, the doxorubicin-containing composition of the present invention (Composition 1 in example 1) and the conventional doxorubicin formulation (aqueous solution of doxorubicin hydrochloride, Free-Dox) were tested on the human uterine cancer cell lines, MES-SA (doxorubicin-sensitive cell line) and MES-SA/Dx-5 (doxorubicin-resistant cell line).

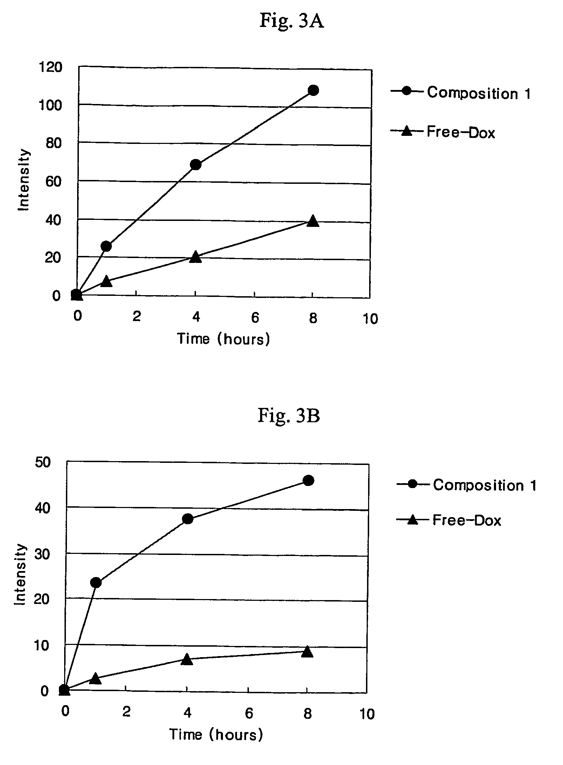

The flow cytometry study was performed with the FACStarPlus (Becton Dickinson) according to the method of Walker et al. (Experimental Cell Research 207: 142(1993)). Briefly, cells(1.0 x 10⁶) were incubated in McCoy's 5A medium (Invitrogen Corp.) supplemented with 10% fetal bovine serum and 1% penicillin streptomycin. After a 24-hour incubation period, the cells were treated with the drug composition at a dose of 1.0 µg/ml. Cells in 12 x 75 Falcon tubes were placed on the FACStarPlus and the fluorescence was observed at a wavelength of 488 nm(excitation) and 519 nm(emission). Data were analyzed by CellQuest software and the results are shown in Figs. 3A to 4B and summarized in Tables 2A and 2B.

**Table 2A: Number of cells(%) which absorbed the drug**

| Time (hr) | MES-SA | | | MES-SA/Dx-5 | | |
|---|---|---|---|---|---|---|
| | Free-Dox | Comp.1 | Ratio* | Free-Dox | Comp.1 | Radio* |
| 0 | 1.0 | 1.0 | 1.0 | 1.0 | 1.0 | 1.0 |
| 1 | 4.0 | 16.3 | 4.0 | 1.8 | 10.4 | 5.8 |
| 4 | 11.3 | 68.9 | 6.1 | 3.2 | 22.7 | 7.1 |
| 8 | 35.1 | 92.3 | 2.6 | 5.3 | 29.3 | 5.5 |

| | | | | | | |
|---|---|---|---|---|---|---|
| *Ratio = Composition 1 / Free-Dox | | | | | | |

**Table 2B: Change of (Δ) Fluorescence Intensity**

| Time (hr) | MES-SA | | | MES-SA/Dx-5 | | |
|---|---|---|---|---|---|---|
| | Free-Dox | Comp.1 | Ratio* | Free-Dox | Comp.1 | Ratio* |
| 0 | 0.0 | 0.0 | - | 0.0 | 0.0 | - |
| 1 | 7.6 | 25.6 | 3.4 | 2.6 | 23.4 | 9.0 |
| 4 | 20.6 | 68.6 | 3.3 | 7.1 | 37.6 | 5.3 |
| 8 | 40.6 | 108.3 | 2.7 | 9.2 | 46.2 | 5.0 |

| | | | | | | |
|---|---|---|---|---|---|---|
| *Ratio = Composition 1 / Free-Dox | | | | | | |

As shown in Figs. 2A to 3B and Tables 2A and 2B, the drug enters more effectively into the cells from the drug composition of the present invention (Composition 1) than from the conventional solution formulation (Free-Dox). Particularly the uptake of doxorubicin into cells is remarkable in the drug resistant cell line.

### Example 12A

### Confocal microscopy: doxorubicin-containing composition in MES-SA cells

To visualize the intracellular uptake of a bioactive agent, the doxorubicin-containing composition of the present invention (Composition 1 in Example 1) and the conventional doxorubicin formulation (aqueous solution of doxorubicin hydrochloride, Free-Dox) were tested on the human uterine cancer cell lines, MES-SA (doxorubicin-sensitive cell line) and MES-SA/Dx-5 (doxorubicin-resistant cell line).

The cells were imaged on a Zeiss (Thornwood,NY) LSM 510 confocal imaging system with an inverted fluorescence microscope and an image analyzer. Briefly, cells(3 x 105) in 3ml of McCoy's 5A medium (Invitrogen Corp.) supplemented with 10% fetal bovine serum and 1% penicillin streptomycin were incubated overnight on a glass coverslip at 37°C. After incubation, cells were treated with the drug composition at a dose of 1.0 µg/ml, and the coverslip was mounted on the microscope. At given time intervals, the fluorescence was observed at an excitation wavelength of 484nm, and the results are shown in Figs. 4A to 4D.

The confocal images in Figs. 4A to 4D visualize the flow cytometry results: much higher amounts of doxorubicin were absorbed by the cells when the doxorubicin containing composition of the present invention was treated. In Figs. 4A to 4D, the left side pictures are the confocal images after treatment with the conventional solution formulation and the images on the right side are after treatment with the compositions of the present invention. And as shown in Figs. 4B and 4D, the micelles or nanoparticles were detected in the cytoplasmic and nuclear compartments.

### Example 12B

### Confocal microscopy: epirubicin-containing composition in MCF-7 cells

To visualize the intracellular uptake of a drug, an epirubicin-containing composition of the present invention (Composition 6 in Example 6) and the conventional epirubicin formulation (aqueous solution of epirubicin hydrochloride) were tested on the human breast cancer cell lines, MCF-7 (epirubicin-sensitive cell line) and MCF-7/ADR (epirubicin-resistant cell line).

The confocal images were obtained by the same procedure described in example 12A, using RPMI medium (Invitrogen Corp.) instead of McCoy's 5A medium, and the results are shown in Figs. 4E to 4H.

The confocal images in Figs. 4E to 4H visualize the flow cytometry results: much higher amounts of epirubicin were absorbed by the cells when the epirubicin containing composition of the present invention was treated. In Figs. 4E to 4H, the left side pictures are the confocal images after treatment with the conventional solution formulation and the images on the right side are after treatment with the compositions of the present invention. And as shown in Figs. 4F and 4H, the micelles or nanoparticles were detected in the cytoplasmic and nuclear compartments.

### Example 13

### In vitro cytotoxicity

For the in vitro cytotoxicity test of the composition of the present invention, the doxorubicin-containing composition of the present invention (Composition 1 in Example 1, Doxo-PNP) and the conventional doxorubicin formulation (aqueous solution of doxorubicin hydrochloride, Free-Dox) were tested on the human uterine cancer cell lines, MES-SA (doxorubicin-sensitive cell line) and MES-SA/Dx-5 (doxorubicin-resistant cell line). MTT assay is well established method for cytotoxicity test. When the cells are treated with MTT (methylthiazoletetrazolium), the MTT-formazan is produced from reduction of MTT-tetrazolium by enzymes present in living cells only(dead cells cannot reduce the MTT-tetrazolium to MTT-formazan). The fluorescence of the MTT-formazan is detected by a fluorescence reader and the optical density correlates with the number of cells. This procedure is automatized, and the cell viability and IC₅₀ (50% inhibitory concentration of cell growth) values are calculated by the software installed in the microplate reader.

The cytotoxic activity of each composition was evaluated in both human tumor cell lines at five ten-fold dilutions ranging from 0.01 to 100 µg/ml. Following continuous exposure for 3 days, the cells were treated with MTT (methylthiazoletetrazolium). The MTT-formazan produced from reduction of MTT-tetrazolium by enzymes present in the living cells was detected by a fluorescence reader. The optical density correlates with the number of cells. The results of two independent experiments were expressed as IC₅₀ (50% inhibitory concentration) values of each cell line. The MTT assay was performed essentially according to the method of Carmichael et al. (Cancer Research 47: 936(1987)). Briefly, cells were harvested from an exponential phase culture growing in McCoy's 5A medium (Invitrogen Corp.) supplemented with 10% fetal bovine serum and 1% penicillin streptomycin, counted and plated in 96 well flat-bottomed microtiter plates (100 cell suspension, 5 x 10⁴ cells/ml for each cell line). After a 24h recovery to allow the cells to resume exponential growth, a culture medium (24 control wells per plate) or culture medium containing drug was added to the wells. Each drug concentration was plated in triplicate. Following 3 days of continuous drug exposure, the cells were treated with 25 µℓ of a MTT solution in sterile water (2mg/ml). Fluorescence was measured using an automatic microplate reader (SpectraMax 190, Molecular Devices) at a wavelength of 549nm, and the number of viable cells was calculated from the optical density.

The results of the cell viability study are shown in Figs. 5A and 5B and IC₅₀ (50% inhibitory concentration) values of each cell line are summarized in Table 3.

**Table 3: MTT Assay (IC₅₀, µg/ml)**

| Time (hr) | MES-SA | | | MES-SA/Dx-5 | | |
|---|---|---|---|---|---|---|
| | Free-Dox | Comp.1 | Ratio* | Free-Dox | Comp.1 | Ratio* |
| 24 | 0.30 | 0.14 | 2.1 | 54.0 | 0.95 | 56.8 |
| 48 | 0.087 | 0.068 | 1.3 | 0.92 | 0.28 | 3.3 |
| 72 | 0.031 | 0.018 | 1.7 | 0.75 | 0.14 | 5.4 |

| | | | | | | |
|---|---|---|---|---|---|---|
| * Ratio = Free-Dox / Composition 1 | | | | | | |

The cytotoxic activity on the doxorubicin-sensitive cells was similar in both compositions as shown in Figs. 5A, but the drug composition of the present invention showed 6.7 times higher activity at three days after treatment than the conventional solution formulation when treating the doxorubicin-resistant cells as shown in Fig. 5B. This difference in activity is due to the characteristics of the drug-resistant cell lines in which the P-glycoproteins (P-gp) are overexpressed and they continuously extrude the cytotoxic drugs from the cell. Since free drug cannot be concentrated within the drug-resistant cells, this result implies that the drug carrier of the present invention enters together the cell with the drug incorporated in the drug carrier.

### Example 14

### Pharmacokinetics in rats

The drug concentrations in blood plasma were measured after intravenous administration of the doxorubicin-containing compositions in the present invention (Compositions 1 to 5 in examples 1 to 5) and the conventional doxorubicin formulation (aqueous solution of doxorubicin hydrochloride, Free-Dox) in 7- to 8-week old Sprague-Dawley rats (200~250g).
The rats (5 rats for each formulation) were injected intravenously through the tail vein at a dose of 5mg/kg. The blood samples were collected from the tail vein at 1, 5, 15, 30 minutes and 1, 2, 4, 8, and 24 hours after the drug injection. The blood samples were immediately centrifuged, and the plasma was separated. The plasma samples were stored at -50 °C until analysis. Doxorubicin was analyzed by HPLC assay described in example 1. The blood concentration-time curve(C-t curve) is shown in Fig. 6, and the area under the blood concentration-time curve (AUC) was calculated using the linear trapezoidal rule. The results are summarized in Table 4.

**Table 4: Pharmacokinetics in Rats**

| | Concentration (µg·hr/mL) | | | | AUC (µg·hr/mL) |
|---|---|---|---|---|---|
| | 1hr | 4hr | 8hr | 24hr | |
| Free-Dox | 0.23 | 0.07 | 0.04 | 0.03 | 1.3 |
| Composition 1 | 7.41 | 4.69 | 3.85 | 1.99 | 85.6 |
| Composition 2 | 5.23 | 2.59 | 1.57 | 0.85 | 42.0 |
| Composition 3 | 1.47 | 0.85 | 0.63 | 0.31 | 14.7 |
| Composition 4 | 2.10 | 1.35 | 0.95 | 0.60 | 23.2 |
| Composition 5 | 3.54 | 2.35 | 1.96 | 1.16 | 44.2 |

## Claims

1. The use of an intracellular delivery system in the manufacture of a medicament for use in the delivery of at least one bioactive agent within a cell,
wherein the intracellular delivery system comprises at least one bioactive agent and a polymeric drug carrier with said bioactive agent entrapped therein in the aqueous solution, and wherein the polymeric drug carrier is prepared by a polymeric composition comprising:
(a) an amphiphilic block copolymer consisting of a hydrophilic block and a hydrophobic block in which said hydrophobic block has a terminal hydroxyl group that is substituted with a tocopherol or cholesterol group, and
(b) a polylactic acid derivative having at least one carboxyl group at the end of the polymer;
and wherein said bioactive agent entrapped in the polymeric drug carrier is at least one selected from the group consisting of nucleic acids, proteins and polypeptides, with the proviso that the polypeptide is not cyclosporine, and is allowed to be delivered into a cell in a greater quantity when the drug carrier contacts with said cell.

2. The use of claim 1 wherein said tocopherol or cholesterol group comprises a tocopherol succinic acid or cholesterol succinic acid group, and said polylactic acid derivative has at least one carboxyl group at the end of the polymer and 0.01 to 10 equivalents of a di- or tri-valent metal ion with respect to 1 equivalent of the carboxyl terminal group of the polylactic acid derivative.

3. The use of Claim 2, wherein said di- or tri-valent metal ion is one selected from the group consisting of Ca²⁺, Mg²⁺ , Ba²⁺, Cr³⁺, Fe³⁺, Mn²⁺, Ni²⁺, Cu²⁺, Zn²⁺ and Al³⁺.

4. The use of Claim 1, 2 or 3, wherein said polylactic acid derivative is represented by the following formula:
RO-CHZ-[A]ₙ[B]ₘ-COOM (I)
wherein A is -COO-CHZ-; B is -COO-CHY-, -COO-CH₂CH₂CH₂CH₂CH₂- or -COO-CH₂CH₂OCH₂; R is a hydrogen atom, acetyl, benzoyl, decanoyl, palmitoyl, methyl or ethyl group; Z and Y each are hydrogen atoms, methyl, or phenyl groups; M is H, Na, K, or Li; n is an integer from 1 to 30, and m is an integer from 0 to 20.

5. The use of Claim 1, 2 or 3, wherein said polylactic acid derivative is represented by the following formula:
RO-CHZ-[COO-CHX]ₚ-[COO-CHY']_{q}-COO-CHZ-COOM (II)
wherein X is a methyl group; Y' is hydrogen atom or phenyl group; p is an integer from 0 to 25; q is an integer from 0 to 25, provided that p+q is an integer from 5 to 25; R is a hydrogen atom, acetyl, benzoyl, decanoyl, palmitoyl, methyl or ethyl group; Z is a hydrogen atom, methyl, or phenyl group; and M is H, Na, K, or Li.

6. The use of Claim 1, 2 or 3, wherein said polylactic acid derivative is represented by the following formula:
RO-PAD-COO-W-M' (III)
wherein W-M' is 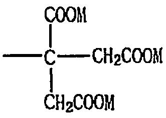 or 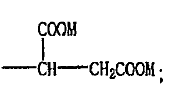 PAD is a member selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-Lactic acid and caprolactone, and a copolymer of D,L-lactic acid and 1,4-dioxan-2-one; R is a hydrogen atom, acetyl, benzoyl, decanoyl, palmitoyl, methyl or ethyl group; and M is H, Na, K, or Li.

7. The use of Claim 1, 2 or 3, wherein said polylactic acid derivative is represented by the following formula:
S-O-PAD-COO-Q (IV)
wherein S is 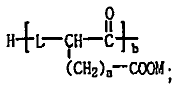 L is -NR₁- or -O-; R₁ is a hydrogen atom or C₁₋₁₀alkyl; Q is CH₃, CH₂CH₃, CH₂CH₂CH₃, CH₂CH₂CH₂CH₃, or CH₂C₆H₅; a is an integer from 0 to 4; b is an integer from 1 to 10; R is a hydrogen atom, acetyl, benzoyl, decanoyl, palmitoyl, methyl or ethyl group; M is H, Na, K, or Li and PAD is a member selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-Lactic acid and caprolactone, and a copolymer of D,L-lactic acid and 1,4-dioxan-2-one.

8. The use of Claim 1, 2 or 3, wherein said polylactic acid derivative is represented by the following formula: 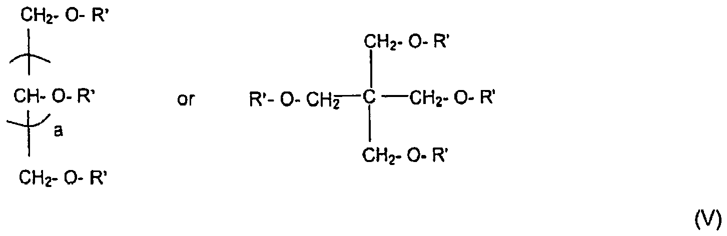 wherein R' is -PAD-O-C(O)-CH₂CH₂-C(O)-OM and PAD is a member selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-Lactic acid and caprolactone, and a copolymer of D,L-lactic acid and 1,4-dioxan-2-one.; M is the same as defined in formula (I); a is an integer from 1 to 4.

9. Use of an intracellular delivery system in the manufacture of a medicament for use in the delivery of at least one bioactive agent within a cell,
wherein the intracellular delivery system comprises at least one bioactive agent and a polymeric drug carrier with said bioactive agent entrapped therein in the aqueous solution, and wherein the polymeric drug carrier is prepared by a polymeric composition comprising
(a) an amphiphilic block copolymer consisting of a hydrophilic block and a hydrophobic block in which said hydrophobic block has a terminal hydroxyl group that is substituted with a tocopherol or cholesterol group, and
(b) a polylactic acid derivative having at least one carboxyl group at the end of the polymer;
and wherein said bioactive agent entrapped in the polymeric drug carrier is at least one selected from the group consisting of epirubicin and doxorubicin, and allowed to be delivered into a cell in a greater quantity when the drug carrier contacts with said cell, and wherein said polylactic acid derivative is represented by any one selected from the following formulae:
RO-CHZ-[A]ₙ-[B]ₘ-COOM
wherein A is -COO-CHZ-; B is -COO-CHY-, -COO-CH₂CH₂CH₂CH₂CH₂- or -COO-CH₂CH₂OCH₂-; R is a hydrogen atom, acetyl, benzoyl, decanoyl, palmitoyl, methyl or ethyl group; Z and Y each are hydrogen atoms, or methyl groups; M is H, Na, K, or Li; n is an integer from 1 to 30, and m is an integer from 0 to 20;
RO-CHZ-[COO-CHX]ₚ-[COO-CHY']_{q}-COO-CHZ-COOM
wherein X is a methyl group; Y' is hydrogen atom; p is an integer from 0 to 25; q is an integer from 0 to 25, provided that p + q is an integer from 5 to 25; R is a hydrogen atom, acetyl, benzoyl, decanoyl, palmitoyl, methyl or ethyl group; Z is a hydrogen atom, or methyl group; and M is H, Na, K, or Li;
RO-PAD-COO-W-M
wherein W-M' is 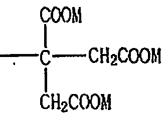 or 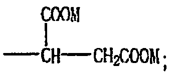 PAD is a member selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-Lactic acid and caprolactone, and a copolymer of D,L-lactic acid and 1,4-dioxan-2-one; R is a hydrogen atom, acetyl, benzoyl, decanoyl, palmitoyl, methyl or ethyl group; and M is H, Na, K, or Li;
S-O-PAD-COO-Q
wherein S is 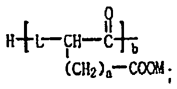 L is -NR¹- or -O-; R¹ is a hydrogen atom or C₁₋₁₀ alkyl; Q is CH₃, CH₂CH₃, CH₂CH₂CH₃, CH₂CH₂CH₂CH₃, or CH₂C₆H₅; a is an integer from 0 to 4; b is an integer from 1 to 10; R is a hydrogen atom, acetyl, benzoyl, decanoyl, palmitoyl, methyl or ethyl group; M is H, Na, K, or Li and PAD is a member selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-Lactic acid and caprolactone, and a copolymer of D,L-lactic acid and 1,4-dioxan-2-one; and 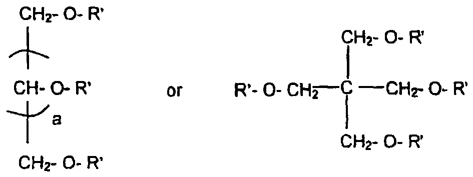 wherein R' is -PAD-O-C(O)-CH₂CH₂-C(O)-OM and PAD is a member selected from the group consisting of D,L-polylactic acid, D-polylactic acid, polymandelic acid, a copolymer of D,L-factic acid and glycolic acid, a copolymer of D,L-lactic acid and mandelic acid, a copolymer of D,L-Lactic acid and caprolactone, and a copolymer of D,L-lactic acid and 1,4-dioxan-2-one.; M is the same as defined in formula (I); a is an integer from 1 to 4.

10. The use of Claim 9, wherein said tocopherol or cholesterol group comprises a tocopherol succinic acid or cholesterol succinic acid group, and said a polylactic acid derivative has at least one carboxyl group at the end of the polymer and 0.01 to 10 equivalents of the di- or tri-valent metal ion with respect to 1 equivalent of the carboxyl terminal group of the polylactic acid derivative.

11. The use of Claim 10, wherein said di- or tri-valent metal ion is one selected from the group consisting of Ca²⁺, Mg²⁺, Ba²⁺, Cr³⁺, Fe³⁺, Mn²⁺, Ni²⁺, Cu²⁺, Zn²⁺ and Al³⁺.

12. The use of any one of Claims 1 to 11, wherein said hydrophilic block is one selected from the group consisting of polyalkylene glycols, polyvinyl pyrrolidone, polyvinyl alcohols and polyacryl amides, and the hydrophobic block is one selected from the group consisting of polylactides, polyglycolides, polydioxan-2-one, polycaprolactone, polylactic-co-glycolide, polylactic-co-caprolactone, polylactic-co-dioxan-2-one, and derivatives thereof wherein the carboxyl terminal group is substituted with a tocopherol succinic acid or cholesterol succinic acid group.

13. The use of any preceding Claim, wherein said hydrophilic and hydrophobic blocks have a number average molecular weight within the range of 500 to 50,000 g/mole, respectively.

14. The use of any preceding Claim, wherein the ratio of the hydrophilic block to the hydrophobic block in the amphiphilic block copolymer is 3:7 to 8:2.

15. The use of any preceding Claim, wherein said polylactic acid derivative has a number average molecular weight of 500 to 2,500 g/mole.

16. The use of any preceding Claim, wherein said polylactic acid derivative is in the form of a sodium or potassium salt obtained by a condensation reaction in the absence of a catalyst followed by neutralization with sodium carbonate, sodium hydrogen carbonate, potassium hydrogen carbonate, or potassium carbonate.

17. The use of any preceding Claim, wherein said polymeric composition comprises 0.1 to 99.9 wt% of the amphiphilic block copolymer and 0.1 to 99.9 wt% of the polylactic acid derivatives based on the total weight of the amphiphilic block copolymer and the polylactic acid derivative.

18. The use of any preceding Claim, wherein the ratio of the bioactive agent to the polymeric composition is 0.1-20.0:80.0-99.9 by weight ratio.

19. The use of any preceding Claim, wherein the particle size of the drug carrier is within the range of 1 to 400 nm.

20. The use of any preceding Claim, wherein said drug carrier is polymeric micelle or nanoparticle.

## Patentansprüche

1. Verwendung eines intrazellulären Abgabesystems bei der Herstellung eines Medikaments zur Verwendung bei der Abgabe mindestens eines bioaktiven Wirkstoffs innerhalb einer Zelle,
wobei das intrazelluläre Abgabesystem mindestens einen bioaktiven Wirkstoff und einen polymeren Arzneimittel-Carrier in der wässrigen Lösung umfasst, wobei der genannte bioaktive Wirkstoff in diesem eingeschlossen ist, und wobei der polymere Arzneimittel-Carrier durch eine Polymerzusammensetzung dargestellt wird, welche Folgendes umfasst:
(a) ein amphiphiles Block-Copolymer, welches aus einem hydrophilen Block und einem hydrophoben Block besteht, wobei der genannte hydrophobe Block eine terminale Hydroxylgruppe aufweist, welche mit einer Tocopherol- oder Cholesteringruppe substituiert ist, und
(b) ein Derivat der Polymilchsäure, welches mindestens eine Carboxylgruppe am Ende des Polymers aufweist;
und wobei der genannte in dem polymeren Arzneimittel-Carrier eingeschlossene bioaktive Wirkstoff mindestens ein aus der Gruppe bestehend aus Nukleinsäuren, Proteinen und Polypeptiden ausgewählter ist, mit der Bedingung, dass das Polypeptid nicht Cyclosporin ist, und seine Abgabe in eine Zelle in einer größeren Menge zugelassen wird, wenn der Arzneimittel-Carrier mit der genannten Zelle in Berührung kommt.

2. Verwendung nach Anspruch 1, wobei die genannte Tocopherol- oder Cholesteringruppe eine Tocopherol-Succinat-Gruppe oder Cholesterin-Succinat-Gruppe umfasst und das genannte Derivat der Polymilchsäure mindestens eine Carboxylgruppe am Ende des Polymers aufweist sowie 0,01 bis 10 Äquivalente eines di- oder trivalenten Metallions gegenüber 1 Äquivalent der terminalen Carboxylgruppe des Derivats der Polymilchsäure.

3. Verwendung nach Anspruch 2, wobei das genannte di- oder trivalente Metallion ein aus der Gruppe bestehend aus Ca²⁺, Mg²⁺, Ba²⁺, Cr³⁺, Fe³⁺, Mn²⁺, Ni²⁺, Cu²⁺, Zn²⁺ und Al³⁺ ausgewähltes ist.

4. Verwendung nach Anspruch 1, 2 oder 3, wobei das genannte Derivat der Polymilchsäure durch die folgende Formel:
RO-CHZ-[A]ₙ-[B]ₘ-COOM (I)
dargestellt wird, wobei A gleich -COO-CHZ- ist; B gleich -COO-CHY-, - COO-CH₂CH₂CH₂CH₂CH₂- oder -COO-CH₂CH₂OCH₂ ist; R ein Wasserstoffatom, Acetyl-, Benzoyl-, Decanoyl-, Palmitoyl-, Methyl- oder Ethylgruppe ist; Z und Y jeweils Wasserstoffatome, Methyl- oder Phenylgruppen sind; M gleich H, Na, K oder Li ist; n eine Ganzzahl von 1 bis 30 und m eine Ganzzahl von 0 bis 20 ist.

5. Verwendung nach Anspruch 1, 2 oder 3, wobei das genannte Derivat der Polymilchsäure durch die folgende Formel:
RO-CHZ-[COO-CHX]ₚ-[COO-CHY']_{q}-COO-CHZ-COOM (II)
dargestellt wird, wobei X eine Methylgruppe ist; Y' Wasserstoffatom oder Phenylgruppe ist; p eine Ganzzahl von 0 bis 25 ist; q eine Ganzzahl von 0 bis 25 ist, vorausgesetzt, dass p+q eine Ganzzahl von 5 bis 25 ist; R ein Wasserstoffatom, Acetyl-, Benzoyl-, Decanoyl-, Palmitoyl-, Methyl- oder Ethylgruppe ist; Z ein Wasserstoffatom, Methyl- oder Phenylgruppe ist; und M gleich H, Na, K oder Li ist.

6. Verwendung nach Anspruch 1, 2 oder 3, wobei das genannte Derivat der Polymilchsäure durch die folgende Formel:
RO-PAD-COO-W-M' (III)
dargestellt wird,
wobei W-M' gleich 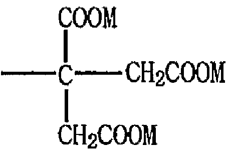 oder 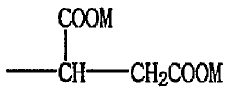 ist;
PAD ein Bestandteil ist, welcher aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on ausgewählt wird; R ein Wasserstoffatom, Acetyl-, Benzoyl-, Decanoyl-, Palmitoyl-, Methyl- oder Ethylgruppe ist; und M gleich H, Na, K oder Li ist.

7. Verwendung nach Anspruch 1, 2 oder 3, wobei das genannte Derivat der Polymilchsäure durch die folgende Formel:
S-O-PAD-COO-Q (IV)
dargestellt wird,
wobei S gleich 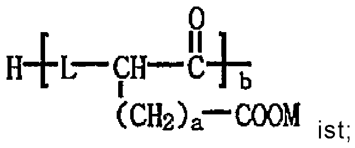 L gleich -NR₁- oder -O- ist; R₁ ein Wasserstoffatom oder C₁₋₁₀alkyl ist; Q gleich CH₃, CH₂CH₃, CH₂CH₂CH₃, CH₂CH₂CH₂CH₃ oder CH₂C₆H₅ ist; a eine Ganzzahl von 0 bis 4 ist; b eine Ganzzahl von 1 bis 10 ist; R ein Wasserstoffatom, Acetyl-, Benzoyl-, Decanoyl-, Palmitoyl-, Methyl- oder Ethylgruppe ist; M gleich H, Na, K oder Li ist und PAD ein Bestandteil ist, welcher aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on ausgewählt wird.

8. Verwendung nach Anspruch 1, 2 oder 3, wobei das genannte Derivat der Polymilchsäure durch die folgende Formel: 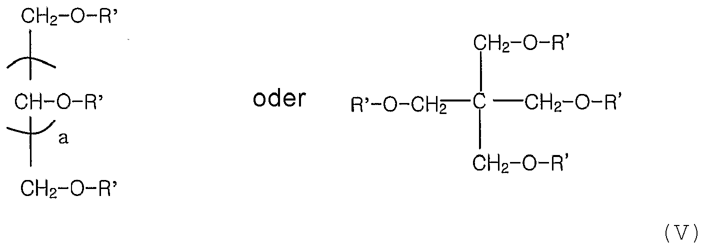 dargestellt wird,
wobei R' gleich -PAD-O-C(O)-CH₂CH₂-C(O)-OM ist und PAD ein Bestandteil ist, welcher aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on ausgewählt wird; M wie in Formel (1) definiert ist; a eine Ganzzahl von 1 bis 4 ist.

9. Verwendung eines intrazellulären Abgabesystems bei der Herstellung eines Medikaments zur Verwendung bei der Abgabe mindestens eines bioaktiven Wirkstoffs innerhalb einer Zelle,
wobei das intrazelluläre Abgabesystem mindestens einen bioaktiven Wirkstoff und einen polymeren Arzneimittel-Carrier in der wässrigen Lösung umfasst, wobei der genannte bioaktive Wirkstoff in diesem eingeschlossen ist, und wobei der polymere Arzneimittel-Carrier durch eine Polymerzusammensetzung dargestellt wird, welche Folgendes umfasst:
(a) ein amphiphiles Block-Copolymer, welches aus einem hydrophilen Block und einem hydrophoben Block besteht, wobei der genannte hydrophobe Block eine terminale Hydroxylgruppe aufweist, welche mit einer Tocopherol- oder Cholesteringruppe substituiert ist, und
(b) ein Derivat der Polymilchsäure, welches mindestens eine Carboxylgruppe am Ende des Polymers aufweist;
und wobei der genannte in dem polymeren Arzneimittel-Carrier eingeschlossene bioaktive Wirkstoff mindestens ein aus der Gruppe bestehend aus Epirubicin und Doxorubicin ausgewählter ist und seine Abgabe in eine Zelle in einer größeren Menge zugelassen wird, wenn der Arzneimittel-Carrier mit der genannten Zelle in Berührung kommt, und wobei das genannte Derivat der Polymilchsäure durch eine beliebige Formel, ausgewählt unter den folgenden Formeln, dargestellt wird:
**RO-CHZ-[A]ₙ-[B]ₘ-COOM**
wobei A gleich -COO-CHZ- ist; B gleich -COO-CHY-, -COO-CH₂CH₂CH₂CH₂CH₂- oder -COO-CH₂CH₂OCH₂ ist; R ein Wasserstoffatom, Acetyl-, Benzoyl-, Decanoyl-, Palmitoyl-, Methyl- oder Ethylgruppe ist; Z und Y jeweils Wasserstoffatome oder Methylgruppen sind; M gleich H, Na, K oder Li ist; n eine Ganzzahl von 1 bis 30 und m eine Ganzzahl von 0 bis 20 ist;
RO-CHZ-[COO-CHX]ₚ-[COO-CHY']_{q}-COO-CHZ-COOM
wobei X eine Methylgruppe ist; Y' Wasserstoffatom ist; p eine Ganzzahl von 0 bis 25 ist; q eine Ganzzahl von 0 bis 25 ist, vorausgesetzt, dass p+q eine Ganzzahl von 5 bis 25 ist; R ein Wasserstoffatom, Acetyl-, Benzoyl-, Decanoyl-, Palmitoyl-, Methyl- oder Ethylgruppe ist; Z ein Wasserstoffatom oder Methylgruppe ist; und M gleich H, Na, K oder Li ist;
**RO-PAD-COO-W-M'**
wobei W-M' gleich 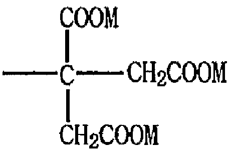 oder 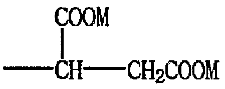 ist;
PAD ein Bestandteil ist, welcher aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on ausgewählt wird; R ein Wasserstoffatom, Acetyl-, Benzoyl-, Decanoyl-, Palmitoyl-, Methyl- oder Ethylgruppe ist; und M gleich H, Na, K oder Li ist;
**S-O-PAD-COO-Q**
wobei S gleich 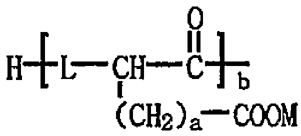 ist;
L gleich -NR¹- oder -O- ist; R¹ ein Wasserstoffatom oder C₁₋₁₀alkyl ist; Q gleich CH₃, CH₂CH₃, CH₂CH₂CH₃, CH₂CH₂CH₂CH₃ oder CH₂C₆H₅ ist; a eine Ganzzahl von 0 bis 4 ist; b eine Ganzzahl von 1 bis 10 ist; R ein Wasserstoffatom, Acetyl-, Benzoyl-, Decanoyl-, Palmitoyl-, Methyl- oder Ethylgruppe ist; M gleich H, Na, K oder Li ist und PAD ein Bestandteil ist, welcher aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on ausgewählt wird; und 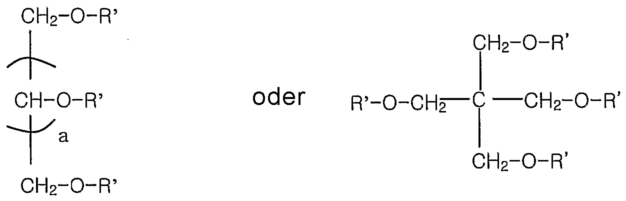 wobei R' gleich -PAD-O-C(O)-CH₂CH₂-C(O)-OM ist und PAD ein Bestandteil ist, welcher aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on ausgewählt wird; M wie in Formel (I) definiert ist; a eine Ganzzahl von 1 bis 4 ist.

10. Verwendung nach Anspruch 9, wobei die genannte Tocopherol- oder Cholesteringruppe eine Tocopherol-Succinat-Gruppe oder Cholesterin-Succinat-Gruppe umfasst und das genannte Derivat der Polymilchsäure mindestens eine Carboxylgruppe am Ende des Polymers aufweist sowie 0,01 bis 10 Äquivalente eines di- oder trivalenten Metallions gegenüber 1 Äquivalent der terminalen Carboxylgruppe des Derivats der Polymilchsäure.

11. Verwendung nach Anspruch 10, wobei das genannte di- oder trivalente Metallion ein aus der Gruppe bestehend aus Ca²⁺, Mg²⁺, Ba²⁺, Cr³⁺, Fe³⁺, Mn²⁺, Ni²⁺, Cu²⁺, Zn²⁺ und Al³⁺ ausgewähltes ist.

12. Verwendung nach einem beliebigen der Ansprüche 1 bis 11, wobei der genannte hydrophile Block ein aus der Gruppe bestehend aus Polyalkylenglykolen, Polyvinylpyrrolidon, Polyvinylalkoholen und Polyacrylamiden ausgewählter ist und der hydrophobe Block ein aus der Gruppe bestehend aus Polylactiden, Polyglykoliden, Polydioxan-2-on, Polycaprolacton, Polylactid-co-Glykolid, Polylactid-co-Caprolacton, Polylactid-co-Dioxan-2-on und Derivaten hiervon ausgewählter ist, wobei die terminale Carboxylgruppe mit einer Tocopherol-Succinat- oder Cholesterin-Succinat-Gruppe substituiert ist.

13. Verwendung nach einem beliebigen vorangehenden Anspruch, wobei der genannte hydrophile und hydrophobe Block jeweils ein zahlenmittleres Molekulargewicht im Bereich von 500 bis 50.000 g/mol aufweist.

14. Verwendung nach einem beliebigen vorangehenden Anspruch, wobei das Verhältnis des hydrophilen Blocks zum hydrophoben Block in dem amphiphilen Block-Copolymer 3:7 bis 8:2 beträgt.

15. Verwendung nach einem beliebigen vorangehenden Anspruch, wobei das genannte Derivat der Polymilchsäure ein zahlenmittleres Molekulargewicht von 500 bis 2.500 g/mol aufweist.

16. Verwendung nach einem beliebigen vorangehenden Anspruch, wobei das genannte Derivat der Polymilchsäure in Form eines Natrium- oder Kaliumsalzes vorliegt, welches durch eine Kondensationsreaktion in Abwesenheit eines Katalysators, gefolgt von einer Neutralisierung mit Natriumcarbonat, Natriumhydrogencarbonat, Kaliumhydrogencarbonat oder Kaliumcarbonat, erhalten wird.

17. Verwendung nach einem beliebigen vorangehenden Anspruch, wobei die genannte polymere Zusammensetzung 0,1 bis 99,9 Gewichtsprozent des amphiphilen Block-Copolymers und 0,1 bis 99,9 Gewichtsprozent der Derivate der Polymilchsäure bezogen auf das Gesamtgewicht des amphiphilen Block-Copolymers und des Derivats der Polymilchsäure umfasst.

18. Verwendung nach einem beliebigen vorangehenden Anspruch, wobei das Verhältnis des bioaktiven Wirkstoffs zu der polymeren Zusammensetzung 0,1-20,0:80,0-99,9 nach Gewichtsverhältnis beträgt.

19. Verwendung nach einem beliebigen vorangehenden Anspruch, wobei die Partikelgröße des Arzneimittel-Carriers im Bereich von 1 bis 400 nm liegt.

20. Verwendung nach einem beliebigen vorangehenden Anspruch, wobei der genannte Arzneimittel-Carrier polymere Mizelle oder Nanopartikel ist.

## Revendications

1. Utilisation d'un système d'administration intracellulaire dans la fabrication d'un médicament destiné à être utilisé dans l'administration d'au moins un agent bioactif à l'intérieur d'une cellule,
dans laquelle le système d'administration intracellulaire comprend au moins un agent bioactif et un support de médicament polymère avec ledit agent bioactif piégé à l'intérieur dans la solution aqueuse et dans laquelle le support de médicament polymère est préparé par une composition polymère comprenant :
(a) un copolymère à blocs amphiphile consistant en un bloc hydrophile et un bloc hydrophobe, dans lequel ledit bloc hydrophobe a un groupe hydroxyle terminal qui est substitué par un groupe tocophérol ou cholestérol ; et
(b) un dérivé d'acide polylactique ayant au moins un groupe carboxyle à l'extrémité du polymère ;
et dans laquelle ledit agent bioactif piégé dans le support de médicament polymère est au moins l'un choisi dans le groupe consistant en acides nucléiques, protéines et polypeptides, à la condition que le polypeptide ne soit pas la cyclosporine, et est amené à être administré dans une cellule dans une quantité supérieure lorsque le support de médicament entre en contact avec ladite cellule.

2. Utilisation selon la revendication 1, dans laquelle ledit groupe tocophérol ou cholestérol comprend un groupe tocophérol acide succinique ou cholestérol acide succinique, et ledit dérivé d'acide polylactique a au moins un groupe carboxyle à l'extrémité du polymère et 0,01 à 10 équivalents d'un ion métallique di- ou trivalent par rapport à 1 équivalent du groupe terminal carboxyle du dérivé d'acide polylactique.

3. Utilisation selon la revendication 2, dans laquelle ledit ion métallique di- ou trivalent est l'un choisi dans le groupe consistant en Ca²⁺, Mg²⁺, Ba²⁺, Cr³⁺, Fe³⁺, Mn²⁺, Ni²⁺, Cu²⁺, Zn²⁺ et Al³⁺.

4. Utilisation selon l'une des revendications 1, 2 ou 3, dans laquelle ledit dérivé d'acide polylactique est représenté par la formule suivante :
**RO-CHZ-[A]ₙ-[B]ₘ-COOM** **(I)**
dans laquelle A est -COO-CHZ- ; B est -COO-CHY-, -COO-CH₂CH₂CH₂CH₂CH₂- ou -COO-CH₂CH₂OCH₂ ; R est un atome d'hydrogène, un groupe acétyle, benzoyle, décanoyle, palmitoyle, méthyle ou éthyle ; Z et Y représentent chacun des atomes d'hydrogène, des groupes méthyle ou phényle ; M représente H, Na, K ou Li ; n est un entier de 1 à 30 et m est un entier de 0 à 20.

5. Utilisation selon l'une des revendications 1, 2 ou 3, dans laquelle ledit dérivé d'acide polylactique est représenté par la formule suivante :
**RO-CHZ-[COO-CHX]ₚ-[COO-CHY']_{q}-COO-CHZ-COOM** **(II)**
dans laquelle X représente un groupe méthyle ; Y' représente un atome d'hydrogène ou un groupe phényle ; p représente un entier de 0 à 25 ; q représente un entier de 0 à 25, à la condition que p+q soit un entier de 5 à 25 ; R représente un atome d'hydrogène, un groupe acétyle, benzoyle, décanoyle, palmitoyle, méthyle ou éthyle ; Z représente un atome d'hydrogène, un groupe méthyle ou phényle ; et M représente H, Na, K ou Li.

6. Utilisation selon l'une des revendications 1, 2 ou 3, dans laquelle ledit dérivé d'acide polylactique est représenté par la formule suivante :
**RO-PAD-COO-W-M'** **(III)**
dans laquelle W-M' représente 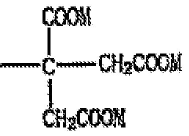 ou 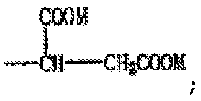 PAD est un élément choisi dans le groupe consistant en l'acide D,L-polylactique, l'acide D-polylactique, l'acide polymandélique, un copolymère de l'acide D,L-lactique et de l'acide glycolique, un copolymère de l'acide D,L-lactique et de l'acide mandélique, un copolymère de l'acide D,L-lactique et de la caprolactone et un copolymère de l'acide D,L-lactique et de la 1,4-dioxan-2-one ; R est un atome d'hydrogène, un groupe acétyle, benzoyle, décanoyle, palmitoyle, méthyle ou éthyle ; et M représente H, Na, K ou Li.

7. Utilisation selon l'une des revendications 1, 2 ou 3, dans laquelle ledit dérivé d'acide polylactique est représenté par la formule suivante :
**S-O-PAO-COO-Q** **(IV)**
dans laquelle S représente 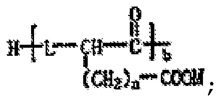 L représente -NR₁- ou -O- ; R₁ représente un atome d'hydrogène ou un alkyle en C₁₋₁₀ ; Q représente CH₃, CH₂CH₃, CH₂CH₂CH₃, CH₂CH₂CH₂CH₃ ou CH₂C₆H₅ ; a est un entier de 0 à 4 ; b est un entier de 1 à 10 ; R représente un atome d'hydrogène, un groupe acétyle, benzoyle, décanoyle, palmitoyle, méthyle ou éthyle ; M représente H, Na, K ou Li et PAD représente un élément choisi dans le groupe consistant en l'acide D,L-polylactique, l'acide D-polylactique, l'acide polymandélique, un copolymère d'acide D,L-lactique et d'acide glycolique, un copolymère d'acide D,L-lactique et d'acide mandélique, un copolymère d'acide D,L-lactique et de caprolactone et un copolymère d'acide D,L-lactique et de 1,4-dioxan-2-one.

8. Utilisation selon l'une des revendications 1, 2 ou 3, dans laquelle ledit dérivé d'acide polylactique est représenté par la formule suivante : 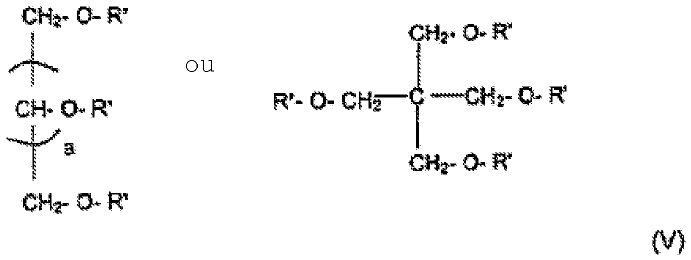 dans lesquelles R' représente -PAD-O-C(O)-CH₂CH₂-C(O)-OM et PAD est un élément choisi dans le groupe consistant en l'acide D,L-polylactique, l'acide D-polylactique, l'acide polymandélique, un copolymère d'acide D,L-lactique et d'acide glycolique, un copolymère d'acide D,L-lactique et d'acide mandélique, un copolymère d'acide D,L-lactique et de caprolactone et un copolymère d'acide D,L-lactique et de 1,4-dioxan-2-one ; M est le même que celui défini dans la formule (I) ; a est un entier de 1 à 4.

9. Utilisation d'un système d'administration intracellulaire dans la fabrication d'un médicament destiné à être utilisé dans l'administration d'au moins un agent bioactif à l'intérieur d'une cellule,
dans laquelle le système d'administration intracellulaire comprend au moins un agent bioactif et un support de médicament polymère avec ledit agent bioactif piégé à l'intérieur dans la solution aqueuse et dans laquelle le support de médicament polymère est préparé par une composition polymère comprenant
(a) un copolymère à blocs amphiphile consistant en un bloc hydrophile et un bloc hydrophobe dans lequel ledit bloc hydrophobe a un groupe hydroxyle terminal qui est substitué par un groupe tocophérol ou cholestérol ; et
(b) un dérivé d'acide polylactique ayant au moins un groupe carboxyle à l'extrémité du polymère ;
et dans laquelle ledit agent bioactif piégé dans le support de médicament polymère est au moins l'un choisi dans le groupe consistant en l'épirubicine et la doxorubicine, et amené à être administré dans une cellule dans une quantité supérieure lorsque le support de médicament entre en contact avec ladite cellule et dans laquelle ledit dérivé d'acide polylactique est représenté par l'une quelconque choisie parmi les formules suivantes :
**RO-CHZ-[A]ₙ-[B]ₘ-COOM**
dans laquelle A représente -COO-CHZ- ; B représente -COO-CHY-, -COO-CH₂CH₂CH₂CH₂CH₂- ou -COO-CH₂CH₂OCH₂- ; R représente un atome d'hydrogène, un groupe acétyle, benzoyle, décanoyle, palmitoyle, méthyle ou éthyle ; Z et Y représentent chacun des atomes d'hydrogène ou des groupes méthyle ; M représente H, Na, K ou Li ; n est un entier de 1 à 30 et m est un entier de 0 à 20 ;
**RO-CHZ-[COO-CHX]ₚ-[COO-CHY]_{q}-COO-CHZ-COOM**
dans laquelle X représente un groupe méthyle ; Y' représente un atome d'hydrogène ; p est un entier de 0 à 25 ; q est un entier de 0 à 25, à condition que p+q soit un entier de 5 à 25 ; R représente un atome d'hydrogène, un groupe acétyle, benzoyle, décanoyle, palmitoyle, méthyle ou éthyle ; Z représente un atome d'hydrogène ou un groupe méthyle ; et M représente H, Na, K ou Li ;
**RO-PAD-COO-W-M**
dans laquelle W-M' représente 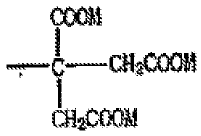 ou 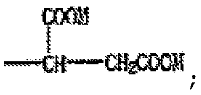 PAD est un élément choisi dans le groupe consistant en l'acide D,L-polylactique, l'acide D-polylactique, l'acide polymandélique, un copolymère d'acide D,L-lactique et d'acide glycolique, un copolymère d'acide D,L-lactique et d'acide mandélique, un copolymère d'acide D,L-lactique et de caprolactone et un copolymère d'acide D,L-lactique et de 1,4-dioxan-2-one ; R représente un atome d'hydrogène, un groupe acétyle, benzoyle, décanoyle, palmitoyle, méthyle ou éthyle ; et M représente H, Na, K ou Li ;
**S-O-PAD-COO-Q**
dans laquelle S représente 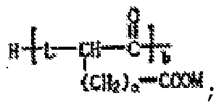 L représente -NR¹- ou -O- ; R¹ représente un atome d'hydrogène ou un alkyle en C₁₋₁₀ ; Q représente CH₃, CH₂CH₃, CH₂CH₂CH₃, CH₂CH₂CH₂CH₃ ou CH₂C₆H₅ ; a est un entier de 0 à 4 ; b est un entier de 1 à 10 ; R représente un atome d'hydrogène, un groupe acétyle, benzoyle, décanoyle, palmitoyle, méthyle ou éthyle ; M représente H, Na, K ou Li, et PAD est un élément choisi dans le groupe consistant en l'acide D,L-polylactique, l'acide D-polylactique, l'acide polymandélique, un copolymère d'acide D,L-lactique et d'acide glycolique, un copolymère d'acide D,L-lactique et d'acide mandélique, un copolymère d'acide D,L-lactique et de caprolactone et un copolymère d'acide D,L-lactique et de 1,4-dioxan-2-one ; et 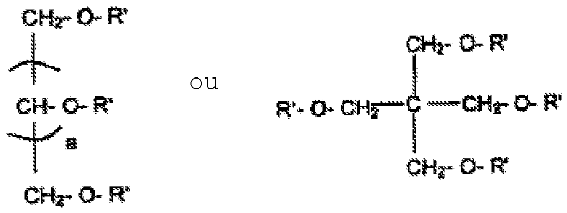 dans lesquelles R' représente -PAD-O-C(O)-CH₂CH₂-C(O)-OM et PAD est un élément choisi dans le groupe consistant en l'acide D,L-polylactique, l'acide D-polylactique, l'acide polymandélique, un copolymère d'acide D,L-lactique et d'acide glycolique, un copolymère d'acide D,L-lactique et d'acide mandélique, un copolymère d'acide D,L-lactique et de caprolactone et un copolymère d'acide D,L-lactique et de 1,4-dioxan-2-one ; M est le même que celui défini dans la formule (I) ; a est un entier de 1 à 4.

10. Utilisation selon la revendication 9, dans laquelle ledit groupe tocophérol ou cholestérol comprend un groupe tocophérol acide succinique ou cholestérol acide succinique et ledit dérivé d'acide polylactique a au moins un groupe carboxyle à l'extrémité du polymère et 0,01 à 10 équivalents de l'ion métallique di- ou trivalent par rapport à 1 équivalent du groupe terminal carboxyle du dérivé d'acide polylactique.

11. Utilisation selon la revendication 10, dans laquelle ledit ion métallique di- ou trivalent est l'un choisi dans le groupe consistant en Ca²⁺, Mg²⁺, Ba²⁺, Cr³⁺, Fe³⁺, Mn²⁺, Ni²⁺, Cu²⁺, Zn²⁺ et Al³⁺.

12. Utilisation selon l'une quelconque des revendications 1 à 11, dans laquelle ledit bloc hydrophile est l'un choisi dans le groupe consistant en les polyalkylène glycols, la polyvinyl pyrrolidone, les alcools polyvinyliques et les polyacrylamides, et le bloc hydrophobe est l'un choisi dans le groupe consistant en les polylactides, les polyglycolides, la polydioxan-2-one, la polycaprolactone, le polylactique-co-glycolide, la polylactique-co-caprolactone, la polylactique-co-dioxan-2-one et leurs dérivés dans lesquels le groupe carboxyle terminal est substitué par un groupe tocophérol acide succinique ou cholestérol acide succinique.

13. Utilisation selon l'une quelconque des revendications précédentes, dans laquelle lesdits blocs hydrophile et hydrophobe ont une masse moléculaire moyenne en nombre se situant respectivement dans la plage de 500 à 50000 g/mole.

14. Utilisation selon l'une quelconque des revendications précédentes, dans laquelle le rapport du bloc hydrophile au bloc hydrophobe dans le copolymère à blocs amphiphile est de 3:7 à 8:2.

15. Utilisation selon l'une quelconque des revendications précédentes, dans laquelle ledit dérivé d'acide polylactique a une masse moléculaire moyenne en nombre de 500 à 2500 g/mole.

16. Utilisation selon l'une quelconque des revendications précédentes, dans laquelle ledit dérivé d'acide polylactique se présente sous la forme d'un sel de sodium ou de potassium obtenu par une réaction de condensation en l'absence d'un catalyseur suivie par une neutralisation par le carbonate de sodium, l'hydrogéno carbonate de sodium, l'hydrogéno carbonate de potassium ou le carbonate de potassium.

17. Utilisation selon l'une quelconque des revendications précédentes, dans laquelle ladite composition polymère comprend 0,1 à 99,9 % en poids du copolymère à blocs amphiphile et 0,1 à 99,9 % en poids des dérivés d'acide polylactique sur la base du poids total du copolymère à blocs amphiphile et du dérivé d'acide polylactique.

18. Utilisation selon l'une quelconque des revendications précédentes, dans laquelle le rapport de l'agent bioactif à la composition polymère est de 0,1-20,0 : 80,0-99,9 en rapport de poids.

19. Utilisation selon l'une quelconque des revendications précédentes, dans laquelle la dimension de particule du support de médicament se situe dans la plage de 1 à 400 nm.

20. Utilisation selon l'une quelconque des revendications précédentes, dans laquelle ledit support de médicament est une micelle polymère ou une nanoparticule polymère.
